# EUROPEAN PATENT APPLICATION

(11) **EP 1 878 439 A2**
(43) Date of publication of application: **16.01.2008**
(21) Application number: 07016087.4
(22) Date of filing: 08.12.2003
(51) Int. Cl.: A61K 39/102, C07K 14/285, C07K 16/12, C12N 5/10, C12N 15/31, G01N 33/50

(54) **Actinobacillus pleuropneumoniae virulence genes**

(30) Priority: 09.12.2002 GB 0228691
(62) Divisional of application: 03767972.7
(71) Applicant: Imperial Innovations Limited, London SW7 2AZ (GB)
(72) Inventor: Kroll, John S., Exhibition Road London SW7 2BT (GB); Langford, Paul R., Exhibition Road London SW7 2BT (GB); Bosse, Janine, Exhibition Road London SW7 2BT (GB); Beddek, Amanda, Exhibition Road London SW7 2BT (GB); Rycroft, Andrew, Exhibition Road London SW7 2BT (GB); Sheenan, Brian, Exhibition Road London SW7 2BT (GB)
(74) Representative: Griffin, Philippa Jane

(57) **Abstract**

The application provides an attenuated *Actinobacillus pleuropneumoniae* bacterium having a mutation in a gene required for bacterial virulence, wherein said gene comprises the nucleotide sequence SEQ ID NO: 10. The application also provides an attenuated *Actinobacillus pleuropneumoniae* bacterium having a mutation in a nucleic acid sequence responsible for, or involved in, controlling expression of a gene required for bacterial virulence, wherein said nucleic acid sequence comprises the nucleotide sequence SEQ ID NO: 47.

## Description

The present invention relates to the identification of genes involved in the virulence of *Actinobacillus pleuropneumoniae,* to the production of attenuated mutant strains of *Actinobacillus pleuropneumoniae,* and to the identification of anti-bacterial agents which target the identified genes and their products.

*A. pleuropneumoniae* is the causative agent of porcine pleuropneumonia, a highly contagious, respiratory disease of pigs. The disease is often fatal and as a result leads to severe economic loss in the swine producing industry. An obligate parasite of the porcine respiratory tract, the incidence of infection of A. *pleuropneumoniae* has increased in recent years, notably where animals are subjected to intensive breeding conditions. Transmission of the bacterium is by aerosol, or by direct contact with infected pigs.

Pleuropneumonia can occur in swine of all ages, and there are no known associations with predisposing viral or bacterial infections. *A. pleuropneumoniae* infection may be chronic or acute, with the disease being characterised by increasingly severe pulmonary distress, which progresses rapidly to death, or to a chronic infection resulting in a failure to thrive. A hemorrhagic, necrotic bronchopneumonia with accompanying fibrinous pleuritis is typically observed.

Fifteen different serotypes of *A*. *pleuropneumoniae* have been identified based on antigenic differences in their capsular polysaccharides, and their production of extracellular toxins. Serotypes 1, 5 and 7 are the most common forms of A. *pleuropneumoniae* infection in the United States, whereas serotypes 1, 2, 5, 7 and 9 predominate in Europe.

Treatment of pigs infected with *A. pleuropneumoniae* usually involves the direct injection of an antibiotic (typically tetracycline). Such a method is however labour intensive, time consuming, and expensive, and can often be of limited use due to the rapid progression of the disease. Evidence from field and experimental studies has however shown that infection with *A. pleuropneumoniae* can provide a lifetime protection against subsequent reinfection, thereby suggesting that vaccination might be a feasible alternative to antibiotic therapy. The development of an effective vaccine has so far been hampered by the antigenic diversity of the fifteen different serotypes.

In attempts to produce a vaccine composition, killed, whole cell bacteria were found to provide only serotype-specific protection, but it has been shown that natural infection with a highly virulent serotype can stimulate a strong protective cross-immunity against multiple serotypes [Nielsen, Nord Vet Med. 31: 407-13 (1979); Nielsen, Nord Vet Med. 36: 221-234 (1984); Nielsen, Can J Vet Res. 29: 580-582 (1988); Nielsen, ACTA Vet Scand. 15: 80-89 (1994)]. Certain undefined live attenuated mutants have also shown promise for cross- protection of swine [Inzana et al, Infect Immun. 61: 1682-6 (1993); Paltineanu et al, In International Pig Veterinary Society, 1992, p.214; Utrera et al, In International Pig Veterinary Society, 1992, p.213] but because of the uncertainties associated with vaccines comprising bacterial strains having such undefined spontaneous mutations, there exists a need in the art for the rational construction of live attenuated strains which will safely stimulate protective immunity against homologous, and preferably heterologous *Actinobacillus pleuropneumoniae* serotypes. There also exists a need to identify the genes involved in A. *pleuropneumoniae* virulence, to facilitate the development of methods for identifying anti-bacterial agents which target those genes and/or their products.

Published International patent application WO 00/61724 (Pharmacia & Upjohn) describes the use of signature tagged mutagenesis (STM) to identify genes responsible for the virulence of gram-negative bacteria. The development of vaccines containing attenuated bacteria is also discussed. The work is also described in Fuller et al, Microbial Pathogenesis [2000] vol. 26; pp.39-51.

In the STM technique, a plurality of different bacterial strains, each of which carries a random mutation in its genome, is produced by means of transposon integration.

The inserted transposons carry different DNA signature tags, which allows individual mutants to be differentiated from one other. The tags each comprise a variable central region flanked by invariant "arms", the latter of which allow the variable central portion to be amplified by PCR. Multiple tagged mutant strains are collected in microtiter dishes, and then combined to form an "inoculum pool" for animal infection.

At an appropriate time after infection, bacteria are isolated from the infected animal, and are combined to form a "recovered pool." The tags in the recovered pool and the tags in the inoculum pool are separately amplified, labelled, and used to probe filters arrayed with all the different tags from the mutants in the inoculum. Mutant strains with attenuated virulence are generally those which cannot be recovered from the infected animal, i.e. strains with tags that give hybridization signals when probed with tags from the inoculum pool, but not when probed with tags from the recovered pool. The advantage of STM is that a large number of insertional mutant strains can be simultaneously screened in a single animal for loss of bacterial virulence. STM is more fully described in e.g. WO 96/17951.

After considerable research, the present inventors have provided improved methods and means for identifying genes involved in *A. pleuropneumoniae* virulence. This has led to the identification of a new family of "virulence genes" the members of which each comprise a nucleotide sequence which is selected from any one of those defined by SEQ ID NO: 1-56. As used herein, the term "virulence genes" refers to genes of a bacterium whose correct function is involved in, e.g. required for the establishment and/or maintenance of an infection in a host animal. Virulence genes and their products, which may be polynucleotides (e.g. rRNA) and/or polypeptides, are thus involved in the pathogenesis of the bacterium, but might not be necessary for its growth *in vitro.*

Mutations in virulence genes can result in an "attenuated bacterium", i.e. one whose ability to establish and/or maintain a bacterial infection in a host animal is reduced, as compared to a corresponding wild-type bacterium of the same strain and serotype. Attenuation of the bacterium may be confirmed by means of its administration to a subject animal, where its inability to cause a sustained infection leading to e.g. chronic infection or death may be observed. The non-establishment of symptoms of porcine pleuropneumonia, e.g. of pulmonary distress, hemorrhagic necrotic bronchopneumonia and/or fibrinous pleuritis, may also be used as an index of bacterial attenuation. Attenuated bacteria are of commercial importance in vaccine compositions.

In one aspect, the present invention thus provides an attenuated *Actinobacillus pleuropneumoniae* bacterium.

In a particular embodiment, the present invention provides an attenuated *Actinobacillus pleuropneumoniae* bacterium having a mutation in a gene which comprises a nucleotide sequence selected from the group consisting of SEQ ID NO.:1-56.

The mutation may inhibit or prevent the function of the gene, e.g. by reducing the level of its expression (e.g. by reducing transcription and/or translation), and/or by impairing the biological activity of the gene product, e.g. by causing an inactive form of the gene product that is encoded by the mutated gene to be produced. The gene product may be a polynucleotide or polypeptide.

The mutation in the gene may be an insertion, deletion or substitution. It may be located in a region of the gene that encodes a polypeptide, or in a sequence responsible for, or involved in, the control of gene expression, e.g. in a promoter region. Mutations which involve a combination of one or more of insertion, deletion and/or substitution are also contemplated. Attenuated A. *pleuropneumoniae* strains of the present invention likewise include those which have two or more mutations, each of which may be an insertion, a deletion, a substitution, or a combination thereof. Multiple mutations may occur in a single gene, or they may occur in different genes within the same bacterial genome. They may result in an additive or synergistic level of attenuation. Multiple mutations may be prepared by design, or may fortuitously arise as a result of techniques for introducing a single mutation.

Deletion mutants include those in which all or part of the virulence gene is deleted. In one aspect, the mutation results in the deletion of at least about 10%, at least about 20%, at least about 30%, at least about 40%, at least about 50%, at least about 60%, at least about 70%, at least about 80%, at least about 90%, at least about 95%, at least about 98%, or at least about 99% of the virulence gene. A deletion may occur in the appropriate nucleotide sequence set forth in SEQ ID N0.:1-56, or it may occur in an adjacent region of the gene, e.g. in an associated control sequence.

Deletion mutants can be constructed using any technique which is well known to those skilled in the art. One strategy employs counter-selectable markers, and has been used to delete genes in many different bacteria. For details of this technique, reference may be made to e.g. Reyrat et al, Infection and Immunity [1998] vol. 66; pp.4011-4017, and Oswald et al, FEMS Microbiol Lett [1999] vol. 179; pp.153-160.

The process employs a double selection strategy, in which a plasmid is constructed which encodes both a selectable and a counter-selectable marker, with flanking DNA sequences being derived from both sides of the desired deletion site in the bacterial genome. The selectable marker is used to select for bacteria in which the plasmid has integrated into the genome in the appropriate location and manner. The counter-selectable marker is then used to select for that small percentage of bacteria which spontaneously eliminate the integrated plasmid. A fraction of those resultant bacteria will contain the desired deletion, with no foreign DNA remaining.

In another technique for achieving a deletion, the *cre-lox* system is used for site specific recombination of DNA. The system consists of 34 base pair lox sequences that are recognised by a bacterial cre recombinase. If the *lox* sites are introduced into the genomic DNA in an appropriate orientation, a sequence which is flanked by the lox sites can be excised by expression of the cre recombinase, one copy of the *lox* site being retained. Using standard recombination techniques, it is possible to delete a sequence of interest in the A. *pleuropneumoniae* genome and to replace it with a selectable marker (e.g. a gene encoding kanamycin resistance) which is flanked by *lox* sites. Transient expression of the *cre* recombinase (e.g. from an electroporated suicide (non-replicating) plasmid containing the *cre* gene under the control of a promoter which functions in A. *pleuropneumoniae)* will then result in efficient elimination of the lox flanked marker. This process results in a bacterial mutant containing the desired deletion, and one copy of the lox sequence.

In another approach to providing a deletion, a target sequence in the *A. pleuropneumoniae* genome can be deleted (replaced) with a marker gene, e.g. a green fluorescent protein (GFP), β-galactosidase or luciferase. DNA segments flanking a desired deletion are prepared by PCR and cloned into a suicide vector for *A. pleuropneumoniae.* An expression cassette, containing a promoter active in A. *pleuropneumoniae* and operably linked to the appropriate marker gene, is then cloned between the flanking sequences. The plasmid is introduced into wild-type bacteria and mutants which both incorporate and express the marker gene are isolated and examined for a desired recombination event (replacement of the wild-type gene with the marker gene).

As for insertion mutants, these include genes in which a stop codon is inserted into an open reading frame (ORF) upstream (5') of the native, i.e. endogenous stop signal. A truncated, inactive form of the (polypeptide) virulence gene product may thus be expressed by the gene. Insertions which cause a frame-shift are likewise contemplated for the production of inactive products. Mutated virulence genes of A. *pleuropneumoniae* will also include those in which a transposon has been inserted into a protein-coding or regulatory sequence of the gene. Attenuated bacteria produced by the STM technique are insertional mutants in which a virulence gene has been rendered non-functional by insertion of a transposon sequence, e.g. Tn *10.*

Because insertional mutants, e.g. those generated by STM, will still contain all of the genetic information required for bacterial virulence, there is a risk that they could revert to a pathogenic state, e.g. by spontaneous deletion of the inserted sequence. Accordingly, when preparing a vaccine or other therapeutic composition, based on an insertional mutant, e.g one identified by STM, it may be desirable to take the information obtained from that STM-derived strain, i.e. the identification of the disrupted virulence gene, and then recreate a mutant in which some, most, or all of that virulence gene has instead been deleted. This should preclude the possibility that the bacterium will revert to a virulent state, thereby to cause pathology, rather than prophylactic protection of a host animal to which it is administered.

In a related aspect, the present invention provides a composition containing the attenuated A. *pleuropneumoniae* bacterium according to the first aspect of the invention. The composition may be an immunogenic composition, i.e. one which is capable of eliciting an antibody response in a host animal to which it is administered. The composition may be a therapeutic (e.g. prophylactic) composition. It may be a vaccine, preferably one whose administration to an animal confers a degree of protection against subsequent infection with serotypes and strains of A. *pleuropneumoniae* different from that/those which is/are present in the vaccine itself (cross-protection). The invention further provides for the use of an attenuated A. *pleuropneumoniae* bacterium of the first aspect of the invention for the manufacture of a medicament (e.g. vaccine) for preventing or alleviating an infection of an animal with A. *pleuropneumoniae* and/or for preventing or alleviating symptoms associated with such infection, e.g. for the prophylactic protection of swine against porcine pleuropneumonia.

In order for an attenuated bacterium to be effective in a vaccine composition, the (one or more) mutation(s) in the virulence gene(s) should be significant enough to prevent the pathogen from evoking severe clinical symptoms, but insignificant enough to allow a limited replication and growth of the bacteria in the host animal, thereby to elicit appropriate defence mechanisms resulting in immunological memory. Mutations in genes which are required for (as distinct from merely involved in) virulence are preferred.

Compositions of the invention may contain a plurality of different attenuated *A*. *pleuropneumoniae* bacteria, e.g. bacteria having different mutations in the same virulence gene, and/or bacteria having similar, or different, mutations in two or more different genes. While it is possible for an avirulent (attenuated) bacterium of the present invention to be administered alone, it is preferably administered in a solid or liquid composition containing one or more suitable adjuvants, diluents, carriers, vehicles and/or excipients, as discussed elsewhere herein. Compositions according to, and for use in the present invention, may thus comprise one or more of these substances, which should be pharmacologically "acceptable", in the sense of being both compatible with the attenuated bacterium, and not deleterious to the animal to be immunized. Additional substances are preferably both sterile and pyrogen free.

Examples of adjuvants which may be used in the present invention include oil-based adjuvants such as Freund's Complete Adjuvant and Freund's Incomplete Adjuvant, mycolate-based adjuvants (e.g. trehalose dimycolate), bacterial lipopolysaccharide (LPS), peptidoglycans (e.g. mureins, mucopeptides, or glycoproteins such as N-Opaca, muramyl dipeptide [MDP], or MDP analogs), proteoglycans (e.g. those extracted from *Klebsiella pneumoniae),* streptococcal preparations (e.g. OK432), BiostimTM (e.g. O1K2), the "Iscoms" of EP 109 942, EP 180 564 and EP 231 039, aluminum hydroxide, saponin, DEAE-dextran, neutral oils (such as miglyol), vegetable oils (such as arachis oil), liposomes, Pluronic polyols, the Ribi adjuvant system (see e.g. GB-A-2 189 141) and interleukins, particularly those that stimulate cell-mediated immunity. An adjuvant consisting of extracts of *Amycolata,* a genus of bacteria of the order Actinomycetales, has also been described (US 4,877,612).
Proprietary adjuvant mixtures are commercially available. The adjuvant to be used will depend, at least in part, on the recipient animal. The amount of adjuvant to be administered will also depend on the type and size of the animal. Optimal dosages may be readily determined by routine methods well known to those skilled in the art.

As for any diluent(s), carrier(s), vehicle(s) and/or excipient(s), these may be liquid, semisolid, or solid in nature. Any suitable substance known to those skilled in the art may be used. Exemplary substances include polyoxyethylene sorbitan monolaurate, magnesium stearate, methyl and propylhydroxybenzoate, talc, alginates, starches, lactose, sucrose, dextrose, sorbitol, mannitol, gum acacia, calcium phosphate, mineral oil, cocoa butter, and oil of theobroma.

The compositions of the invention may be packaged in various forms which maybe convenient for, or adapted to, a particular route of delivery. Therapeutic compositions (e.g. vaccines) may be suitable for administration to a subject animal by for example intravenous, intradermal, intramuscular, intramammary, intraperitoneal and/or subcutaneous injection, and/or by oral, sublingual, nasal, anal or vaginal delivery. The treatment may consist of a single dose, or of a plurality of doses over a period of time. Decisions on administration regimes are well within the ambit of those skilled in the art. The composition may be formulated and packaged as appropriate.

In a further aspect, the present invention provides an isolated polynucleotide encoding a gene product which is naturally involved in (e.g. required for) the virulence of A. *pleuropneumoniae.* The sequence of the polynucleotide is thus found in a virulence gene of A *pleuropneumoniae.* The invention also provides for a polynucleotide encoding a gene product which is not naturally found in A. *pleuropneumoniae,* but whose expression therein is capable of modulating (e.g. of decreasing) the virulence of that bacterium. Such a polynucleotide may be introduced into the bacterium to cause such expression (e.g. by transformation, transfection or electroporation). Expression may result in an attenuated strain of the bacterium, e.g. for use in a vaccine or other immunogenic composition. Polynucleotides which are not naturally found in A. *pleuropneumoniae* but which are capable of modulating the virulence of that bacterium by their direct interaction with *A. pleuropneumoniae* virulence genes or gene products (rather than by indirect action through their own gene products) are also contemplated.

Any gene product of a polynucleotide according to the present invention may be a polynucleotide (e.g. RNA) or a polypeptide. Polypeptide gene products are herein referred to as "virulence polypeptides", and are involved in/capable of modulating the virulence of A. *pleuropneumoniae,* whether or not they are naturally encoded by the wild-type bacterial genome. Polynucleotide gene products which can affect the virulence of A, *pleuropneumoniae* include e.g. antisense RNA and ribozymes. An effect on bacterial virulence may be demonstrated for any gene product by determining the extent to which a strain of the bacterium *A. pleuropneumoniae* which expresses the product can establish and/or maintain an infection in an animal as compared with *A. pleuropneumoniae* of the same strain and serotype which does not express the product.

According to this aspect, the present invention more particularly provides for an isolated polynucleotide comprising (e.g. consisting of): (a) a nucleotide sequence selected from the group consisting of SEQ ID NO.: 1-56 (such sequences are found within the virulence genes of A. *pleuropneumoniae* as identified elsewhere herein); (b) a nucleotide sequence encoding the polypeptide which is encoded by the nucleotide sequence recited in (a); (c) a nucleotide sequence which hybridizes to the nucleotide sequence of (a) and/or (b), or to its complement, under conditions of moderate to high stringency; or (d) a fragment of any one of the nucleotide sequences of (a)-(c), which fragment retains an immunological property and/or a biological activity of the recited nucleotide sequence of (a)-(c). Polynucleotides of the present invention may be DNA or RNA, as described elsewhere herein.

Nucleotide sequences of type (b) can be readily identified once an amino acid sequence is derived from the nucleotide sequence of (a), and account is taken of the genetic code.

As to (c), exemplary, conditions of moderate stringency include a final wash in a buffer comprising 2xSSC/0.1 % SDS, at 35°C to 45°C. Exemplary conditions of high stringency include a final wash in a buffer comprising 0.2xSSC/0.1 % SDS, at 65°C to 75°C. It is understood in the art that conditions of equivalent stringency can be achieved by means of variation of temperature and buffer or of salt concentration. In this regard, reference may be made to Ausubel et al, Protocols in Molecular Biology, John Wiley & Sons (1994), pp.6.0.3 to 6.4.10. Modifications in hybridization conditions can be empirically determined or precisely calculated based on length and percentage of guanosine/cytosine (GC) base pairing. Hybridization conditions can be calculated as described in Sambrook et al, Molecular Cloning: A Laboratory Manual, Cold Spring Harbor Laboratory Press: Cold Spring Harbor, New York (1989), pp. 9.47 to 9.51.

For fragments of type (d), knowledge of the nucleotide sequence according to (a), (b) or (c) makes available to the skilled person every possible fragment of that nucleotide sequence. Fragments may be at least 10, at least 15, at least 20, at least 25, at least 30, at least 35, at least 40, at least 45, or at least 50 nucleotides in length. They may be capable of hybridising to the nucleotide sequence of (a), (b) or (c), or to its complement, under conditions of moderate to high stringency, optionally in the presence of competitive nucleic acid, e.g. in the presence of nucleic acid from a cDNA or mRNA library derived from *A. pleuropneumoniae.* The retained immunological property may be an ability of the fragment to be bound specifically by an antibody which binds specifically to the full length nucleotide sequence. Binding affinities of at least 10⁷M⁻¹ are contemplated. A retained biological activity may be an ability of the fragment to encode a polypeptide which shares a binding capability with the polypeptide encoded by the full length nucleotide sequence, e.g an ability to bind to one of the ligands to which it naturally binds *in vivo,* or the same antibody.

As will be readily apparent to the person of skill in the art, the identification of virulence genes of *Actinobacillus pleuropneumoniae* enables the identification of corresponding (i.e. homologous) genes in other related bacteria, e.g. in other members of the *Pasteurellaceae* family. In isolated form, such gene sequences may be encompassed within the claimed polynucleotides comprising (or consisting of) a nucleotide sequence according to type (c) above. Southern hybridization using the nucleotide sequences of type (a) (or fragments thereof) as probes, can identify such related sequences in genomic, cDNA or mRNA libraries from other bacteria. Alternatively, the use of PCR, with primers comprising the A. *pleuropneumoniae* sequences or fragments thereof, can be equally as effective for identifying virulence genes across species boundaries. As a further alternative (which does not rely on hybridisation but still identifies homologous genes) complementation of an A. *pleuropneumoniae* mutant strain with a polynucleotide (e.g. DNA or mRNA) library from another species, can be used to identify genes having the same, or a related biological activity. In short, polynucleotides of the invention, which comprise a nucleotide sequence according to type (c), may be found in, or constitute, virulence genes of other bacteria related to *A. pleuropneumoniae.*

Identification of related virulence genes can lead to the production of an attenuated strain of the other organism by mutation in that gene, and may therefore be used in the production of vaccines and other immunological compositions. In yet further aspects, the present invention thus provides: (1) an attenuated bacterium (e.g. of the *Pasteurellaceae* family) containing a mutation in a gene comprising a nucleotide sequence which is capable of hybridising to any one of the nucleotide sequences defined by SEQ ID NO: 1-56, under conditions of moderate to high stringency; (2) a composition (e.g. a vaccine) containing such a bacterium; and (3) the use of such a bacterium for the manufacture of a medicament (e.g. a vaccine) for the therapeutic treatment or prophylactic protection of an animal against infection by the corresponding wild-type bacterium (or a different strain or serotype thereof).

As explained elsewhere herein, polynucleotides of the present invention of types (a)-(d) may be e.g. DNA, RNA, or peptide nucleic acids, as described for example in Corey, TIBTECH 15: 224-229 (1997). DNA sequences include those derived (extracted or amplified) from genomic DNA, or from cDNA, as well as wholly or partially chemically synthesized nucleotide sequences. Amplification may be effected by any method well known to those skilled in the art, e.g. by PCR.

"Chemically synthesized" as used herein refers to purely chemical, as opposed to enzymatic (e.g PCR-based) methods for producing polynucleotides. "Wholly" synthesized polynucleotides are those produced entirely by chemical means, and "partially" synthesized polynucleotides include those wherein only portions of the resulting sequence are produced by chemical means.

The polynucleotides of the invention, or their gene products, may be ribozymes.

Such polynucleotides may comprise or consist of an (RNA) nucleotide sequence according to type (c) above, i.e. one which is capable of hybridising to any of the nucleotide sequences recited in (a) or (b) under conditions of moderate to high stringency. For a review of ribozyme technology, reference is made to Gibson and Shillitoe, Mol. Biotech. 7: 125137 (1997). A ribozyme can be utilized to inhibit the translation of mRNA (e.g. mRNA transcribed from virulence genes) in a sequence specific manner, e.g. by: (i) the hybridization of a complementary RNA sequence in the ribozyme to the target mRNA; and (ii) cleavage of the hybridized target mRNA through nuclease activity inherent to the ribozyme. The polynucleotides of the invention may therefore be used to inhibit the expression of virulence genes of A. *pleuropneumoniae* or of other related e.g. *Pasteurellaceae* bacteria. Ribozymes can be identified by empirical methods, but they may be specifically designed based on accessible sites on the target mRNA, e.g. on the mRNA transcription product of the nucleotide sequences defined by SEQ ID NO:1-56 (see also Bramlage et al, Trends in Biotech 16: 434-438 (1998)).

Delivery of ribozymes to target cells, in order to control mRNA translation, may be accomplished using delivery techniques well known to those skilled in the art. Exogenous delivery methods can include the use of targeting liposomes or direct local injection. Endogenous methods include the use of viral vectors and of non-viral plasmids.

Certain polynucleotides of the present invention may also be used to modulate the transcription of virulence genes in *A. pleuropneumoniae,* e.g. by means of oligonucleotide-directed triplet helix formation. For a review of this technique, reference is made to Lavrovsky et al, Biochem. Mol. Med. 62: 11-22 (1997). Triplet helix formation is accomplished using sequence specific oligonucleotides which hybridize to double stranded DNA in the major groove (as defined in the Watson-Crick model). Hybridization of such sequence specific oligonucleotides can thereafter modulate the activity of DNA-binding proteins, including, for example, transcription factors and polymerases. Preferred target sequences for hybridization include transcriptional regulatory regions that modulate the expression of virulence genes. Oligonucleotides which are capable of triplet helix formation are also useful for site-specific covalent modification of target DNA sequences in virulence genes. Oligonucleotides useful for covalent modification are coupled to DNA damaging agents as described for example in Lavrovsky, et al, Biochem. Mol. Med. 62: 11-22 (1997).

In yet another aspect, the present invention provides a vector comprising a polynucleotide according to the present invention. The vector may be DNA or RNA in nature. It may contain one or more control sequences operably linked to the polynucleotide, in order to promote its transcription, and optionally enable its translation, in a suitable host cell under appropriate conditions. Vectors may be plasmids, or viral vectors. They may be capable of autonomous replication. Control sequences may be endogenous (i.e. found within the wild-type bacterium from which the polynucleotide of the invention is derived, e.g. *A. pleuropneumoniae)* or they may be exogenous (e.g. a control sequence found in a related bacterium, e.g in another member of the *Pasteurellaceae* family). Control sequences include *inter alia* promoters and transcription terminators.

Host cells containing (e.g. transformed, transfected, or electroporated with) a polynucleotide or vector of the present invention are further contemplated. Such host cells may be prokaryotic or eukaryotic in nature, either stably or transiently transformed, transfected, or electroporated with a polynucleotide or vector of the present invention, in a manner which permits the transcription of the polynucleotide, and optionally enables translation of the resultant mRNA. Host cells of the invention include bacterial, yeast, fungal, invertebrate, and mammalian cells. Viruses containing a polynucleotide or vector of the present invention are also contemplated.

Host cells of the present invention may be useful in methods for the large scale production of virulence polypeptides (where the polynucleotide of the invention encodes a polypeptide sequence). The cells are grown in a suitable culture medium under favourable conditions, and the desired polypeptides are isolated from the cells, or from the medium in which the cells are grown, by any purification technique well known to those skilled in the art, e.g. by capture on an affinity column. Such cells may be a valuable source of immunogen for the development of antibodies which specifically bind to the virulence polypeptide. Any suitable host cell may be used for the expression of a polynucleotide according to the present invention.

For this and other purposes, polynucleotides of the present invention may be manipulated using standard techniques well within the ambit of those skilled in the art, e.g. the techniques described in Sambrook et al, Molecular Cloning: A Laboratory Manual, Cold Spring Harbor Laboratory Press: Cold Spring Harbor, New York (1989). By way of example, the polynucleotides of the invention may be amplified and cloned by e.g. PCR, e.g. using genomic DNA, cDNA or mRNA as a template. For ease of inserting the cloned sequence into vectors, e.g. expression vectors, PCR primers may be chosen so that the PCR-amplified sequence has a restriction enzyme site at the 5' end which precedes the initiation codon ATG, and a restriction enzyme site at the 3' end after the termination codon TAG, TGA, or TAA. If desirable, the codons in the cloned sequence may be changed, without changing the amino acids, according to the codon preference of an intended host cell, e.g. E. *coli,* as described by Grosjean and Fiers, Guide, 18:199-209 (1982) and Konigsberg and Godson, Proc. Natl. Acad. Sci. (USA), 80: 687-691 (1983). Optimization of codon usage may lead to an increase in the expression of the gene product when produced in that host.

If a virulence polypeptide is to be produced extracellularly, e.g. in the periplasm of a host bacterium, or in the culture medium surrounding a host cell, then the polynucleotide of the present invention may be cloned without its initiation codon, and placed into an expression vector behind an appropriate signal sequence. A number of signal sequences from both prokaryotes and eukaryotes are known to function in the secretion of recombinant proteins. During the protein secretion process, the signal peptide may be removed by a signal peptidase to yield the mature protein.

To simplify the purification of virulence polypeptides, a purification tag may be added to the 5' and/or 3' end of the polypeptide encoding sequence. Commonly used purification tags include a stretch of six histidine residues (US Patent Nos. 5,284,933 and 5,310,663), a streptavidin-affinity tag described by Schmidt and Skerra, Protein Engineering, 6: 109-122 (1993), a FLAG peptide (Hopp et al., Biotechnology, 6: 12051210 (1988)), glutathione S-transferase (Smith and Johnson, Gene, 67: 31-40 (1988)), and thioredoxin (LaVallie et al., BiolTechnology, 11: 187-193 (1993)). To remove these tags, a proteolytic cleavage recognition site may be inserted at the fusion junction. Commonly used proteases are factor Xa, thrombin, and enterokinase.

In a further aspect, the present invention provides a method of producing a virulence polypeptide encoded by a polynucleotide of the invention. The method may comprise the steps of: (i) culturing a host cell according to the present invention under conditions which permit the expression of the polypeptide; and (ii) recovering and optionally isolating the expressed polypeptide from the host cell, or from its surrounding medium. Identification of the polynucleotides of the present invention readily makes available their encoded polypeptides, so such polypeptides can alternatively be chemically synthesised.

Polypeptides encoded by polynucleotides of the present invention (and variants of such polypeptides) are a further aspect of the invention. Such polypeptides are "virulence polypeptides", as discussed elsewhere herein. Compositions containing such polypeptides are further contemplated, and these include vaccines and other immunogenic compositions. Such compositions may contain one or more adjuvants, carriers, diluents, vehicles or excipients, as discussed elsewhere herein.

Variants include biologically and/or immunologically active fragments of the polypeptide encoded by a polynucleotide of the invention, e.g. fragments of the polypeptide encoded by the polynucleotide sequence defined in any one of SEQ ID NO.:1-56, fusions of the polypeptides or fragments thereof with e.g. heterologous polypeptides (thereby to produce chimeras), and analogs of the polypeptides, e.g. polypeptides in which one or more amino acids has been deleted, replaced or added: (i) without loss of one or more of the biological activities and/or immunological characteristics specific for the polypeptide; or (ii) with specific disablement of a particular biological activity of the polypeptide. Replacement may occur using conservative amino acids.

Virulence polypeptides having an additional methionine residue at position -1 are contemplated, as are virulence polypeptides having additional methionine and lysine residues at positions -2 and -1. Variants of these types are particularly useful for recombinant protein production in bacterial cell types. The invention also embraces variants having additional amino acid residues which result from the use of specific expression systems. For example, use of commercially available vectors that express a desired polypeptide as a fusion protein with glutathione-S-transferase (GST) provide the desired polypeptide having an additional glycine residue at position -1 after cleavage of the GST component from the desired polypeptide. Variants of polypeptides of the present invention also include those in which a leader or signal sequence has been removed, e.g. a mature form.

Variant polypeptides include those which have at least about 99%, at least about 95%, at least about 90%, at least about 85%, at least about 80%, at least about 75%, at least about 70%, at least about 65%, at least about 60%, at least about 55%, and at least about 50% sequence identity and/or homology with a virulence polypeptide encoded by a nucleotide sequence as defined in any one of SEQ ID NO.:1-56. Percentage "identity" of an amino acid sequence is defined herein as the percentage of amino acid residues in the candidate sequence that are identical with the residues in the virulence polypeptide, after aligning both sequences and introducing gaps, if necessary, to achieve the maximum percent sequence identity, and not considering any conservative substitutions as part of the sequence identity. Percent "homology" of an amino acid sequence with respect to a virulence polypeptide encoded by any one of SEQ ID NO:1-56 refers to the percentage of amino acid residues in the candidate sequence that are identical with the residues in the virulence polypeptide after aligning the two sequences and introducing gaps, if necessary, to achieve the maximum percent sequence identity, and also considering any conservative substitutions as part of the sequence identity.

By conservative substitution is meant exchange of one amino acid for an amino acid of the same type, the types being as follows:

| | |
|---|---|
| Aliphatic Non-polar : | G A P I L V |
| Polar-uncharged sulphur : | C M |
| Polar-uncharged hydroxyl: | S T |
| Polar-charged acidic: | D E N Q |
| Polar-charged basic: | K R H |
| Aromatic: | F W Y |

In a still further aspect, the present invention extends to antibodies which specifically recognize the polynucleotides or polypeptides of the present invention. Antibodies may be monoclonal or polyclonal antibodies. They include chimeric antibodies, e.g. humanized antibodies, e.g. complementary determining region (CDR)-grafted antibodies, as well as fragments of antibodies (which may be chimeric) e.g. Fab, F(ab')₂, Fd and single chain antibodies. They also include compounds having CDR sequences which specifically recognize a polynucleotide or polypeptide of the present invention. The invention also provides anti-idiotype antibodies which specifically bind to antibodies of the present invention.

The term "specific for" indicates that the variable regions of the antibodies of the invention exclusively recognize a target polypeptide or polynucleotide (i.e. they are able to distinguish a single polypeptide or polynucleotide from related polypeptides or polynucleotides). Binding affinities of at least about 10⁷ M⁻¹, at least about 10⁸ M⁻¹, or at least about 10⁹ M⁻¹ are contemplated. That is not to say that the antibodies will not also interact with other proteins (for example, S. *aureus* protein A or other antibodies in ELISA techniques) through interactions with sequences outside the variable region of the antibodies, and in particular, in the constant region of the molecule. Screening assays to determine binding specificity of an antibody of the invention are well known and routinely practised by those skilled in the art. For a comprehensive discussion of such assays, reference may be made to Harlow et al. (Eds), Antibodies. A Laboratory Manual; Cold Spring Harbor Laboratory; Cold Spring Harbor, NY (1988), Chapter 6.

According to a still further aspect of the present invention, materials and methods are provided for identifying an anti-bacterial agent which is capable of modulating the function of an A. *pleuropneumoniae* virulence gene identified by the present invention, or of a homologous gene in a related species. Agents which are capable of modulating the function of such virulence genes are likewise contemplated *per* se. More particularly, methods of the present invention include screening potential agents for their ability to interfere with the expression and/or biological activity in a host bacterium of the gene products encoded by the nucleotide sequences set forth in any one of SEQ ID NO:. 1-56. Agents that interfere with the expression of virulence gene products include (but are not limited to) anti-sense polynucleotides and ribozymes containing nucleotide sequences which are homologous to any one of the nucleotide sequences of SEQ ID NO:.1-56 (such that they bind to its complement). The invention further embraces methods of modulating the transcription of such virulence genes through the use of oligonucleotide-directed triplet helix formation.

Compositions containing an anti-bacterial agent identified in accordance with the present invention are a still further aspect of the present invention, in addition to a method of treating an animal suffering from a *Pasteurellaceae* (e.g. an A. *pleuropneumoniae)* infection by administration of such an anti-bacterial agent or composition. The invention also embraces the use of such an agent or composition for the manufacture of a medicament for the treatment of a *Pasteurellaceae* infection, e.g. an A. *pleuropneumoniae* infection and/or porcine pleuropneumonia.

Agents that interfere with the function of virulence genes encompass: (i) non-functional variants of the virulence gene products which may be capable of out-competing the wild-type product for its physiological binding sites; and (ii) binding partners of the virulence gene products, which sequester and optionally destroy said products. Agents also include allosteric enzyme inhibitors, in the case where the virulence gene product is an enzyme.

Inhibitory agents may be identified by means of high throughput screening assays using the polynucleotides of the present invention or their encoded products (which may be RNA polynucleotides or polypeptides). Where the screen employs polypeptide gene products having enzymatic activity, assays are established which are based on that activity, and potential agents screened for an ability to modulate (e.g. inhibit) the activity. For virulence gene products which exert their function by interacting with a polypeptide or nucleic acid, binding assays are established to measure such interaction, and potential agents are screened for an ability to modulate, e.g. to inhibit the interaction.

In one example of a binding assay, the virulence gene product is immobilized on a solid support, and an interaction with a (physiological) binding partner is assessed in the presence and absence of a putative inhibitor compound. In another example, interaction between the virulence gene product and its binding partner is assessed in a solution assay, both in the presence and absence of a putative inhibitor compound. In both assays, an inhibitor is identified as a compound that decreases binding between the virulence gene product and its binding partner. Assays for use when the virulence gene product and its binding partner are both polypeptides include variations of the di-hybrid assay, wherein an inhibitor of protein/protein interaction is identified by detection of a positive signal in a transformed or transfected host cell, as described for example in published International patent application WO 95/20652.

Candidate inhibitors contemplated for use in the methods of the present invention include compounds selected from libraries of potential inhibitors. A number of different libraries can be used as a source of small molecule modulators, including: (1) chemical libraries; (2) natural product libraries; and (3) combinatorial libraries comprised of random peptides, oligonucleotides or organic molecules.

Chemical libraries consist of structural analogs of known compounds. Natural product libraries are collections of microorganisms, animals, plants, or marine organisms which are used to create mixtures for screening by: (i) fermentation and extraction of broths from soil, plant or marine microorganisms; or (ii) extraction of plants or marine organisms. Natural product libraries are further discussed in e.g. Science 282: 63-68 (1998). As for combinatorial libraries, these are composed of large numbers of peptides, oligonucleotides, or organic compounds as a mixture. They are relatively easy to prepare by traditional automated synthesis methods, PCR, cloning, or proprietary synthetic methods. Of particular interest are peptide and oligonucleotide combinatorial libraries. For a review of combinatorial chemistry libraries, reference may be made to Myers, Curr. Opin. Biotechnol. 8: 701-707 (1997).

Still other candidate inhibitors, which are contemplated for use in the methods of the invention, may be specifically designed. These include soluble forms of the binding partners of virulence polypeptides. The term "binding partners" as used herein encompasses antibodies (although in certain embodiments these are excluded) including fragments and derivatives thereof, and modified compounds comprising antibody domains that specifically bind to the target gene product.

When a polypeptide binding partner for a virulence gene product is not known, assays that identify physiological, or non-physiological and hence sequestering binding partners may be employed, e.g. assays using affinity columns on which the virulence gene product is immobilised. Alternatively, binding interactions between polypeptides may be identified using the yeast two-hybrid system as described in Fields and Song, Nature, 340: 245-246 (1989), and Fields and Sternglanz, Trends in Genetics, 10: 286-292 (1994). Other assays which may be used to search for agents that bind to a polypeptide virulence gene product include that described in US Patent No. 5,585,277, which relies on the principle that proteins generally exist as a mixture of folded and unfolded states, and continually alternate between the two states. When a test ligand binds to the folded form of a target virulence polypeptide, the target protein molecule bound by the ligand remains in its folded state. Thus, the folded target protein is present to a greater extent in the presence of a test ligand which binds the target protein, than in the absence of a ligand. Binding of the ligand to the target protein can be determined by any method which distinguishes between the folded and unfolded states of the target protein. The function of the target protein need not be known in order for this assay to be performed. Virtually any agent can be assessed by this method as a test ligand, including, but not limited to metals, polypeptides, including proteins, lipids, polysaccharides, polynucleotides and small organic molecules.

Another method for identifying ligands of a virulence gene polypeptide is that described in Wieboldt et al, Anal. Chem., 69: 1683-1691 (1997). That technique screens combinatorial libraries of 20-30 agents at a time in solution phase for binding to the target virulence polypeptide. Agents that bind to the target are separated from other smaller library components by centrifugal ultra-filtration. The specifically selected molecules that are retained on the filter are subsequently liberated from the target protein and analysed, e.g. by HPLC and pneumatically assisted electrospray (ion spray) ionization MS. This procedure selects library components with the greatest affinity for the target protein, and is particularly useful for libraries of small molecules.

Binding agents identified by the initial screens maybe evaluated for their effect on virulence *in vivo.* Agents that interfere with bacterial virulence can prevent the establishment of an infection, or reverse the outcome of an infection once it is established. Binding agents may be included in compositions containing pharmaceutically acceptable adjuvants, excipients, diluents, carriers or vehicles, as described elsewhere herein.

Numerous additional aspects and advantages of the invention will now become apparent to those skilled in the art upon consideration of the following detailed description of the invention which describes presently prepared embodiments thereof.

### EXPERIMENT 1

### A. Materials and Methods

### 1. Bacterial strains and growth conditions

A spontaneously nalidixic acid-resistant derivative of the virulent *Actinobacillus pleuropneumoniae* serotype 1 strain 4074 was selected using conventional methods and designated 4074 Nal^{R}. The virulence of this strain was verified by passage in pigs. *A. pleuropneumoniae* strains were propagated at 37°C with 5% CO₂ on brain-heart infusion (BHI) plates supplemented with 10% Levinthal's base (BHI_{L}), or at 37°C in Colombia broth with 5µg/ml β-nicotinamide adenine dinucleotide (NAD) and 11mM CaCl₂.

The E. *coli* strains used in this study were: S17 λ pir [*recA thi pro hsdR⁻M⁺RP4::2-Tc::Mu::Km* Tn7 lysogenized with λ pir phage] as described in Miller and Mekalanos (1988) J. Bacteriol. 170. pp.2575-2583; CC118 λpir[*Δ(are-leu) araDΔlacX74 galE galK phoA20 thi-1 rpsE rpoB argE recA1* lysogenized with λ pir phage] as described in de Lorenzo et al, (1990) J. Bacteriol. 172, pp. 6657-6667; and XL1-Blue *(recA1 endA1 gyrA96 thi-1 hsdR17 supE44 relA1 lac [F'proAB lac*/*^{q} ZΔM15* Tn*10* (Tet')]). *E. coli* strains were maintained in Luria-Bertani (LB) medium with appropriate antibiotics used at the following concentrations: ampicillin 100µg/ml; kanamycin 100µg/ml; nalidixic acid 20µg/ml; tetracycline 10µg/ml.

### 2. DNA manipulations, amplification, labelling, and hybridisation of tags

Chromosomal DNA was isolated from *A. pleuropneumoniae* using a QIAamp mini DNA kit (Qiagen) according to the manufacturer's instructions. Plasmid DNA was isolated from *E. coli* using a Qiagen plasmid midi kit, again according to the manufacturer's instructions. DNA manipulation was performed according to standard techniques as described in Sambrook et al, Molecular Cloning: A Laboratory Manual, Cold Spring Harbor Laboratory Press: Cold Spring Harbor, New York (1989). Probes for southern blots were labelled with [digoxigenin (DIG)-11]-dUTP using a PCR DIG Probe Synthesis Kit (Roche) as described by the manufacturer. Amplification and labelling of DNA signature-tags was performed as described in Hensel et al (1995) Science 269 pp.400-403. Tags were initially amplified from genomic DNA by means of PCR using the following primers:

P6 (5'-CGACTACAACCTCAAGCT-3')

P7 (5'-CGACCATTCTAACCAAGC-3')

The amplified tags were purified on a 2% Nusieve GTG agarose gel and radiolabelled with [³²P]-dCTP in a second PCR, again using P6 and P7. The cycling conditions for both PCRs were as follows: denaturation and enzyme activation 12min at 95°C, followed by 20 cycles of: 95°C for 30s, 50°C for 45s and 72°C for 10s. Reactions were performed using 1 unit of AmpliTaq Gold (Perkin-Elmer) polymerase in a Perkin-Elmer 2400 thermocycler. Prior to use, the invariant flanking DNA was separated from the variable signature-tag by digestion with *HindIII* for 18h at 37°C.

For colony blots, *E. coli* S17 λ pir strains harbouring each of the pLOF/TAG 1-48 plasmids were grown to mid-exponential phase and 100 µl of each culture was transferred to the wells of a microtitre dish. Bacteria were transferred from the microtitre dish to a nylon membrane (Hybond N) using a replica plater (Sigma) and the inoculated membrane placed on the surface of a dried LB agar plate containing ampicillin (100µg/ml). After overnight growth at 37°C, the membranes were treated with 1.5 M NaCl, 0.5 M NaOH for 10min. DNA was fixed to the membrane by microwaving at maximum power for 30s and bacterial debris removed by washing in 2XSSC. Pre-hybridisation and hybridisation were performed at 65°C in Rapid-hyb buffer (Amersham) for 1 h and overnight, respectively. Blots were washed according to standard procedures. To identify potentially attenuated mutants, hybridisation signals on a blot probed with labelled tags prepared from the input pool (inoculum) were compared to identical blots hybridised with labelled tags prepared from the corresponding output pools from at least two different animals.

### 3. Construction of DNA signature-tags and pLOF/TAG 1-48

Double-stranded DNA signature tags were obtained by PCR amplification of the variable oligonucleotide pool RT1 (Hensel et al, 1995 Science 269 pp.400-403) using the following primers:

P3SAL (5'-CGCCATGTCGACCATTCTAACCAAGCTT-3')

P5SAL (5'-CGCCTAGTCGACTACAACCTCAAGCTT-3')

each of which contains recognition sites for the restriction endonuclease *Sall* at its 5' terminus. To facilitate the cloning of the signature-tags in plasmid pLOF/Km, the plasmid was modified to incorporate a unique Sall site. This was done by annealing the following complementary oligonucleotides:

SALTOP (5'-GTCGACCCT-3') and

SALBTM (5'-GTCGACAGG-3')

to give a double-stranded linker containing a *Sall* site flanked by *Sfil*-compatible ends. Plasmid pLOF/Km was digested with *Sfil,* treated with calf intestinal alkaline phosphatase (CIAP), and ligated to the phosphorylated double-stranded linker to give plasmid pLOF/Sal. The PCR-amplified double-stranded DNA signature-tags were digested with *Sall,* gel purified, and cloned into the *Sall-* digested, CIAP-treated, pLOF/Sal to generate a library of uniquely tagged pLOF/TAG plasmids.

Individual transformants in *E. coli* strain CC118 A pir were screened by colony blot hybridisation with their corresponding [³²P]-dCTP-labelled tags to identify 100 tags that amplified and labelled efficiently. These tags were then tested for an absence of cross-hybridisation and 48 plasmids (pLOF/TAG1-48) were selected for further use.

### 4. Construction of the signature-tagged mutant bank

The 48 signature-tagged mini-Tn 10 transposons carried on plasmids pLOF/TAG 1-48 were separately introduced into A. *pleuropneumoniae* by conjugation. A. *pleuropneumoniae* strain 4074 Nal^{R} and *E*. *coli* S17 λ pir harbouring each of the pLOF/TAG 1-48 plasmids were grown overnight at 37°C to the stationary phase of growth. Bacteria were harvested by centrifugation for 15 minutes at 3,000 rpm and washed twice with 10mM MgSO₄. Approximately equal numbers of A. *pleuropneumoniae* recipient and E. *coli* donor cells were mixed, concentrated, and spread onto 0.22µM filters. Filters were placed onto BHI_{L} plates containing 1mM IPTG and incubated at 37°C with 5% CO₂ for 5 hours. Thereafter, the bacteria were removed into 5ml sterile PBS and aliquots plated onto BHI_{L} plates supplemented with nalidixic acid (20µg/ml) and kanamycin (100µg/ml). Four separate matings were performed for each of the 48 signature-tagged mini-Tn*10* transposons. Transconjugant mutants were screened for susceptibility to ampicillin, thereby to eliminate strains carrying co-integrants of the suicide vector, inserted into the chromosome. In total 2064 transposon mutants from 48 individual matings were isolated and arranged into 43 pools of 48 mutants. Mutants were stored in 1.5ml cryotubes with 15% glycerol at -80°C.

### 5. Infection studies

Individual strains in each pool of 48 A. *pleuropneumoniae* signature-tagged mutants were grown separately until the mid-exponential phase of growth. At an OD₆₀₀ of approximately 0.3, the individual mutant strains were combined and the pooled culture was diluted in HEPES to approximately 2 x 10⁶ cfu/ml. Large White Cross piglets, of 6-10 weeks of age and determined to be A. *pleuropneumoniae* free by bacteriological and serological analyses, were inoculated intra-tracheally with 3ml of the diluted culture. The number of cfu in the inoculum was verified by viable counts after plating serial dilutions of the inoculum on selective BHI_{L}. Each mutant pool was used to infect at least two animals.

At approximately 24h post-infection, surviving animals were euthanised and the lungs were removed. Surviving bacteria were recovered from infected lungs by either: (i) extensive lavage with Hanks buffered saline solution (HBSS); or (ii) homogenising the lungs in 500ml HBSS. Aliquots of the bacterial suspension were plated on selective media, and following growth overnight at 7°C with 5% CO₂, approximately 10,000 colonies were pooled (the "output pool") and total chromosomal DNA was prepared.

### 6. Competitive indices

To determine the *in vivo* competitive index (CI) of individual STM mutants, mutant and wild-type bacteria were grown in Colombia broth with 5µg/ml NAD and 11 mM CaCl₂ to an OD₆₀₀ of 0.3. The cultures were then combined to give equal numbers of mutant and wild-type organisms, diluted to 2x10⁶ cfu/ml, and inoculated intra-tracheally into at least two animals. The ratio of mutant to wild-type bacteria in the inoculum was verified by viable counts after plating serial dilutions of the inoculum in parallel on BHI_{L} + 20µg/ml nalidixic acid (mutant and wild-type), and BHI_{L} + 20µg/ml nalidixic acid + 100µg/ml kanamycin (mutantonly). Approximately 24h post-infection, surviving animals were euthanised and the lungs were removed. The lungs were homogenised in 500ml HBSS and aliquots were plated on selective media (see above) to recover surviving bacteria. The *in vivo* Cl was calculated as the output ratio of mutant to wild-type organisms divided by the input ratio of mutant to wild-type organisms. To determine *in vitro* Cls, mutant and wild-type organisms were grown to mid-exponential phase, mixed in equal numbers, and the combined culture incubated, with agitation, at 37°C for a further 3h. Samples were taken at regular intervals, diluted, and plated on selective BHI_{L} as before, and the *in vitro* Cl was calculated as described above.

### 7. Virulence gene identification and DNA sequencing

To identify the *in vivo* attenuating lesions, DNA flanking the site of transposon insertion was cloned or amplified by inverse PCR. For cloning, chromosomal DNA was digested with a panel of restriction enzymes, separated by agarose gel electrophoresis, transferred to a nylon membrane, and probed in a Southern blot with a DIG-labelled probe specific for the Tn*10* kanamycin gene. Suitably sized fragments hybridising to the probe were excised from a gel, purified and cloned into appropriately digested pBR322. Transformants in E. *coli* strain XL1-Blue were selected on LB agar containing 100µg/ml kanamycin.

To facilitate sequencing with the Tn*10* specific oligonucleotide primer:

BS401 (5'-GACAAGATGTGTATCCACC-3')

the cloned insert was further sub-cloned into plasmid pBCKS (Stratagene). Inverse PCR amplification of DNA flanking the site of transposon insertion was performed as described in Fuller, (2000) Microb. Pathog. 29, pp.25-38: chromosomal DNA was digested with Ss*p*l (which cuts once in the transposon), diluted to 150 ng/ml, and self-ligated for 15min using the Rapid DNA Ligation Kit (Roche). The ligated DNA was then amplified using the primer pairs BS401 + TEF48, and BS401 + TEF62 (Fuller, 2000 Microb. Pathog. 29, pp.25-38). Reaction conditions for inverse PCR were as previously described (Fuller, 2000 Microb. Pathog. 29, pp.25-38). The resultant amplicons were purified following agarose gel electrophoresis using a QIAQUICK gel extraction kit (Qiagen). DNA sequencing reactions were performed using the BigDye terminator cycle sequencing kit (Perkin-Elmer). Sequences from each of the attenuated mutants were used to query the non-redundant public DNA and protein databases using the BLAST set of similarity search programs (Altschul et a/1990 J. Mol Biol. 15, pp.403-410).

### B. Results and Discussion

### 1. Generation of signature-tagged mutants of A, pleuropneumoniae.

The plasmid pLOF/Km, which is functional in *A. pleuropneumoniae,* was modified to accept signature-tags by the insertion of a unique *Sal*I site in the mini-Tn10 element. PCR amplified random signature-tags were cloned into the *Sal*I site, and a bank of approximately 30,000 tagged plasmids in *E. coli* CC118 λ pir was obtained. A subset of these transformants was screened by colony blot with their corresponding radiolabelled tags, and signature-tagged plasmids with strong hybridisation signals were selected. Plasmids with cross-hybridising tags were eliminated, and a pool of 48 unique signature-tagged plasmids was assembled. These plasmids (pLOF/TAG 1-48) were transformed into E. *coli* S17 λ pir, and independently transferred to A. *pleuropneumoniae* strain 4074 Nal^{R} by conjugation.

Following the elimination of ampicillin-resistant plasmid cointegrants (0.5-1 %), 2064 transposon mutants from 48 individual matings were isolated, and then arranged into 43 pools of 48 mutants. Southern blot analysis of ten randomly chosen exconjugants with a mini-Tn 10-specific probe confirmed single, random insertion of the tagged transposon (data not shown). However, subsequent sequence analysis of attenuated clones revealed that mini-Tn 10 insertion in *A. pleuropneumoniae* was not entirely random.

### 2. Signature-tagged screen of A. pleuropneumoniae mutants in pigs

Signature-tagged *A. pleuropneumoniae* mutants were screened using a porcine intra-tracheal infection model to identify genes involved in virulence. Bacteria were instilled directly into the trachea as previous studies have shown that aerosol particles produced by sneezing are small enough to penetrate into the lower respiratory tract, obviating the need for colonisation of the upper respiratory tract (Kaltreider HB. in Immunologic and infectious diseases in the lung. (1976) pp. 73-97, Kirkpatrick & Reynolds (Eds) Marcel Dekker, New York).

A range of experimental factors including the infecting dose, and the method of recovery were investigated before we could reproducibly recover the majority of individual mutants following *in vivo* selection in the porcine host. Using challenge doses of 10³, 10⁴, or 10⁵ cfu, for example, there was a poor correlation between the infecting dose and the appearance of clinical or morphological signs of disease. Moreover, when different pigs were infected with the same pool, the hybridisation patterns of the post-passage pool suggested that different clones were missing after passage of a given pool through different animals (data not shown). This inconsistency was overcome by increasing the challenge dose to 10⁶ -10⁷ cfu/pig.

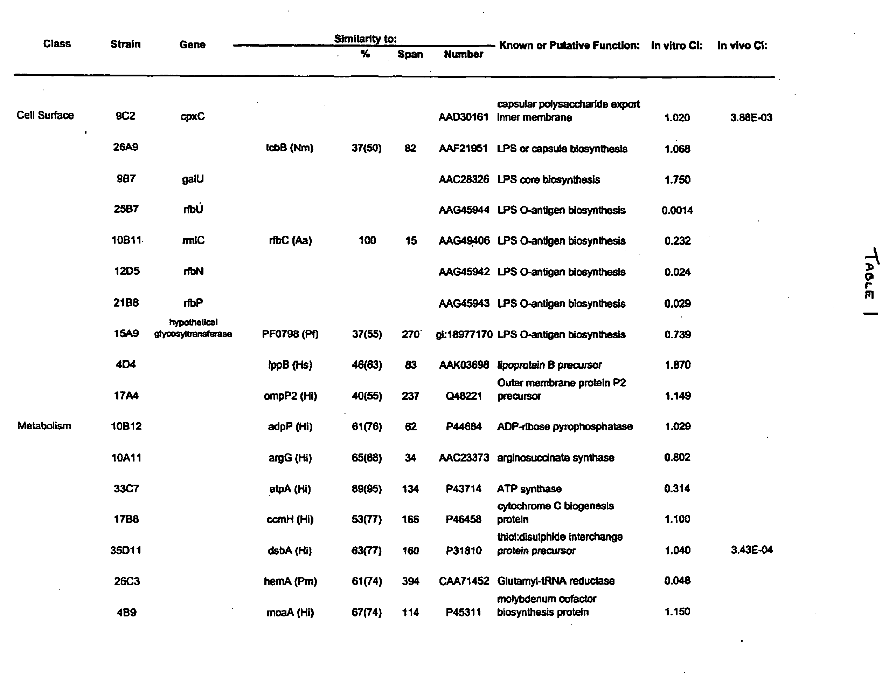

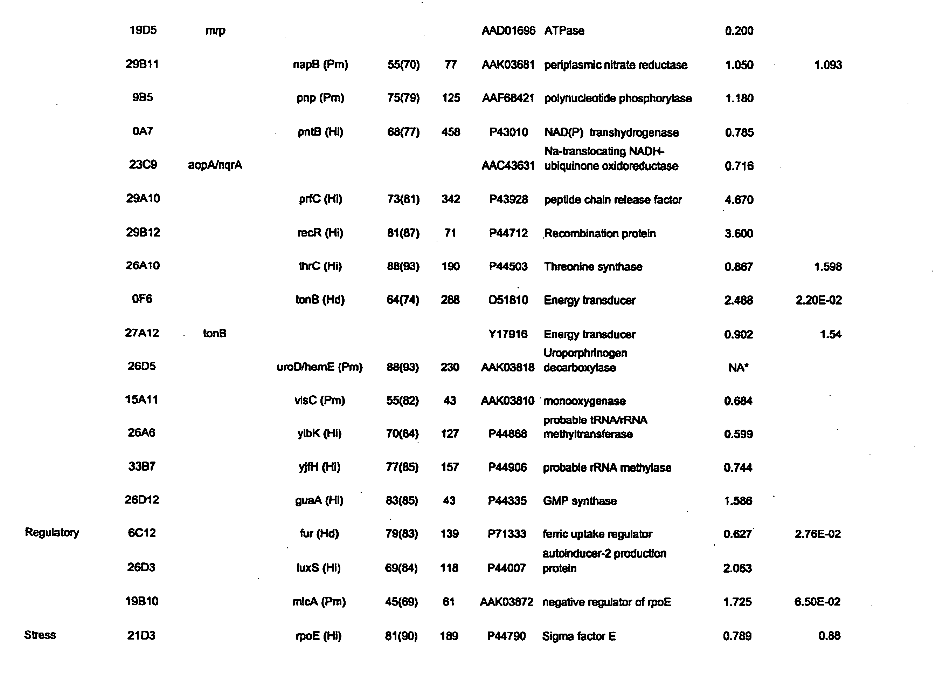

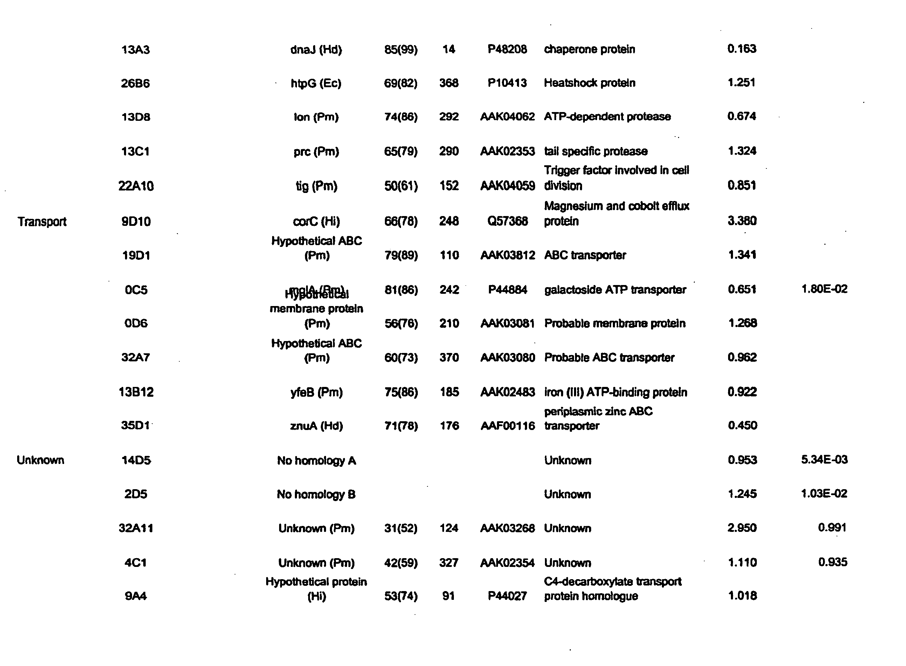

In an earlier STM study of A. *pleuropneumoniae* infection in pigs [Fuller, 2000 Microb. Pathog. 29, pp.39-51], bacteria were recovered by lung lavage following experimental infection. However, this method was found to be unreliable, yielding essentially random recovery of individual signature tags. In contrast, we observed consistent recovery of viable bacteria following homogenisation of the entire lung in HBSS. Optimisation of these two crucial parameters resulted in the recovery of the majority of individual clones (>90%, 20-48h post infection), with excellent reproducibility between animals (see Figure 1; compare A and B).

In total, 2064 transposon mutants (43 pools of 48 mutants) were screened for attenuation *in vivo,* each pool being inoculated into at least two different animals. Surviving bacteria were recovered by homogenisation of lungs in sterile HBSS. This initial screen identified 550 mutants present in the input pool but missing or significantly reduced in the output pools. These potentially attenuated mutants were reassembled into new pools and each new pool was used to infect a further two animals. Following this second screen, 105/410 mutants were found to be consistently absent from the output pools, and these were retained for further analysis.

### 3. Identification and analysis of A. pleuropneumoniae virulence genes.

The nucleotide sequence of the DNA flanking the site of transposon insertion was obtained for each of the 105 attenuated mutants as described, and used to search the GenBank databases for homologous genes. The results of this analysis are shown in Table 1, with the genes assigned to one of five functional categories: cell surface, transport, metabolism, stress responses, and regulation. Insertions in genes encoding proteins with no significant homology to proteins in the databases, or homologous to proteins or predicted proteins of unknown function, are categorised as "unknown". The 105 attenuated mutants contained transposon insertions in 53 individual genes and a number of hotspots for Tn*10* insertion were identified. In particular, seven identical insertions were independently isolated in the putative *rfbP* gene *(Tn* insertion at codon 444),3 of 4 insertions in the A. *pleuropneumoniae fur* gene were located at codon 128. Interestingly, the DNA sequences flanking the 3 hotspots which were identified in this study are neither similar to each other, nor to the consensus sequence for Tn*10* insertion into A. *pleuropneumoniae* that has been previously been identified (Fuller et al (2000) Microb. Pathog. 29, pp.25-38).

### (i) Cell surface

Mutations in capsule and lipopolysaccharide (LPS) genes are known to attenuate *A. pleuropneumoniae* (Rioux et al., Can. J Microbiol. 45: pgs1017-1026, 1999) (Rioux et al., Microb Pathog 28: pgs 279-289, 2000; Ward et al., Infect Immun 66: pgs 3326-3336, 1998). Mutant 9C2 contains a transposon insertion in the *cpxC* gene that encodes a protein involved in capsule export in *A. pleuropneumoniae* (Ward and Inzana, 1997). This mutant was used as a control (known attenuated) strain the *in vivo* Cl experiments. Five genes involved in the synthesis of O-antigen of A. *pleuropneumoniae* serotype 1 were identified in this STM screen. Mutants with inserts in genes 9 (mutant 10B11), 12 (mutant 25B7), 15 (mutant 15A9),17 (mutant 12D5), and 18 (mutant 21B8) of the O-antigen operon (Labrie et al., 2002, J. Endotox Res. 8: 27-38) were all attenuated. Another mutant, 9B7, has the transposon insertion 20 bases upstream of the A. *pleuropneumoniae galU* gene that encodes a protein involved in synthesis of the LPS core. Mutation of *galU* was previously shown to attenuate A. *pleuropneumoniae* (Rioux *et al.,* 1999). Finally, mutant 26A9 contains an insertion in a gene homologous to *IcbB,* a putative bifunctional polymerase implicated in capsule or LPS biosynthesis in *N. meningiditis* serogroup L.

Strain 4D4 contains a transposon insertion in a gene encoding a putative lipoprotein. Strain 4D4 shows greatest homology to an unknown protein from *P. multocida,* but it is similar to lipoproteins and putative lipoproteins of *H. influenzae* and *H. somnus* [Theisen (1993) Infect Immun 61: 1793-1798]. *H. somnus* LppB binds Congo red dye (a structural analogue of heme) and has been suggested to be a virulence factor, but this has not been formally proven [Theisen (1993) Infect lmmun 61:1793-1798]. Finally, strain 17A4 contains an insertion in an ORF with homology to the P2 porin protein of *H. influenzae* [Duim et al (1993) Microb. Pathog. 14, pp. 451-462]. The P2 protein constitutes approximately one half of the total outer membrane protein *of H. influenzae* and represents an important target of the immune response to non-typeable *H. influenzae* infection. [Yi et al (1997) Infect. Immun. 65,150-155].

### (ii) Metabolism

Strain 10B12 contains a transposon insertion in an ADP-ribose pyrophosphatase (*adpP*) gene homologue. Regulation of cellular levels of ADP-ribose (ADP-ribose is derived from NAD) is important in preventing non-enzymatic ADP-ribosylation of proteins. The *E. coli,* ADP-ribose pyrophosphatase catalyses the hydrolysis of ADP-ribose to ribose-5-P and AMP, compounds that can be recycled as part of nucleotide metabolism [Gabelli et al (2001) Nat Struct. Biol. 8, pp. 467-472].

Strain 10A11 carries an insertion in an argininosuccinate synthase, *argG,* gene homologue. Argininosuccinate synthase catalyses the conversion of citrulline and aspartate into argininosuccinate, linking the urea cycle and the citric acid cycle via the so-called aspartate-argininosuccinate shunt. The aspartate-argininosuccinate shunt provides a metabolic link between the pathways by which amino groups and the carbon skeletons of amino acids are processed. (Glansdorff (1996) Biosynthesis of arginine and polyamines. In Escherichia coli and Salmonella (Vol.1) (Neidhardt, F.C. et al., eds), pp. 408-433, ASM Press).

In strain 33C7, the attenuating lesion was found to occur in a gene homologous to *H. influenzae atpA,* encoding the F, alpha subunit of F₁F₀ ATP synthase. ATP synthase is responsible for utilising the proton electrochemical gradient across the cytoplasmic membrane for ATP synthesis in cells growing aerobically or anaerobically. All eight subunits of the bacterial enzyme are encoded by the *atp* operon. In *P. multocida* the *atpA* gene is flanked by *atpH* and *atpG,* and while the gene order in *A. pleuropneumoniae* is unknown, both *atpH* and *atpG* have been implicated previously in the pathogenesis of A. *pleuropneumoniae* infection [Fuller (2000) Microb. Pathog. 29, pp.39-51].

in strain 0A7, the transposon has inactivated the *pntB* gene, encoding the beta subunit of NAD(P) transhydrogenase. Transhydrogenase is an integral membrane protein composed of two subunits, α and β, organised as an α₂β₂ tetramer. Transhydrogenase couples the redox reaction between NAD(H) and NADP(H) to the translocation of protons across the inner membrane (Penfound & Foster (1996) Biosynthesis and recycling of NAD. In Escherichia coli and Salmonella (Vol. 1) (Neidhardt, F.C. et al., eds), pp. 408-433, ASM Press). It plays a key role in energy metabolism, biosynthesis and detoxification.

In strain 19D5, the transposon has inserted in the *A. pleuropneumoniae mrp* gene. The partial A. *pleuropneumoniae mrp* gene sequences available are highly homologous to *mrp* genes from *H. influenzae* and *E. coli.* In *H. influenzae,* the *mrp* gene has been implicated in the biosynthesis of the Gala(1-4)βGal component of LPS, while in E. *coli* mutations in *mrp* affect the synthesis of thiamine [High et al (1996) FEMS Microbiol. Lett. 145, pp.325-331; Petersen (1996) J. Bacteriol. 178, pp. 5676-5682]. In *A. pleuropneumoniae,* the *mrp* gene is located approximately 1 kb upstream from the *apxIVA* gene, encoding the *in vivo*-induced RTX toxin, ApxIV [Schaller (1999) Microbiology 145: 2105-2116].

Strain 29B11 contains a transposon insertion in a *napB* gene homologue, encoding the smaller, dihaem cytochrome c, subunit of heterodimeric periplasmic nitrate reductase (NAP). In *H. influenzae* (and Pseudomonas *sp)* NAP is the sole nitrate reductase and may function to support anaerobic growth in the presence of nitrate [Brige (2001) Biochem. J. 356, pp. 851-858; Fleischmann (1995) Science 269, pp. 496-512; Bedzyk (1999) J Bacteriol 181: 2802-2806]. In *E. coli* the situation is more complex with two membrane bound respiratory nitrate reductases in addition to NAP [Moreno-Vivian (1999) J Bacteriol 181: 6573-6584]. There is evidence to suggest that NAP is a dissimilatory enzyme used for redox balancing. In addition, NAP has been suggested to play a role in adaptation to anaerobic metabolism after transition from aerobic conditions, the utilisation of alternate reductants, or even as a self-defense mechanism forming high nitrite levels to inhibit the growth of competing bacteria [Moreno-Vivian (1999) J Bacteriol 181: 6573-6584]. Consistent with a role in anaerobic adaptation, transcription of the *Salmonella typhimurium napB* gene is induced, in an Fnr-dependent manner, in cells exposed to anaerobic conditions [Wei and Miller (1999) J. Bacteriol. 181, pp. 6092-6097].

Strain 17B8, contains an insertion in a gene homologous to *H. influenzae ccmH,* required for the assembly of C-type cytochromes, including NAP, in enteric bacteria [Thony-Meyer (1995) J. Bacteriol. 177, pp. 4321-4326; Kranz (1998) Mol Microbiol 29, pp. 383-396]. CcmH is encoded by the final gene of the ccm operon and with CcmF and CcmG is believed to catalyse the reduction of disulphide bonds in the cytochrome c apoprotein and to facilitate haem attachment [Kranz (1998) Mol Microbiol 29, pp. 383-396]. While in E. coli the nap and ccm operons are contiguous and may be cotranscribed [Grove (1996) Mol Nicrobiol 19, pp. 467-481] they are located at separate loci in both *H. influenzae* and *P. multocida* [Fleischmann (1995) Science 269, pp. 496-512; May (2001) Proc Natl Acad Sci USA 98, pp. 3460-3465].

Strain 4B9 contains a transposon insertion in a *moaA* gene homologue. In E. *coli* the moaA-E genes are required for the synthesis of molybdopterin from GTP [Rivers (1993) Mol Microbiol 8: 1071-1081]. Molybdopterin (molybdopterin guanine dinucleotide) is the principal cofactor found in prokaryotic molybdoproteins including periplasmic nitrate reductase. Expression of the moa genes is induced by anaerobiosis [Anderson (2000) J. Bacteriol. 182, pp. 7035-7043] and *Salmonella typhi moaA* mutants are impaired in their ability to replicate within epithelial cells [Contreras (1997) Microbiology 143, pp. 2665-2672].

Mutant 29B12 contains an insertion in the A. *pleuropneumoniae recR* gene homologue. The RecR protein is involved in the RecF pathway of recombinatorial DNA repair: *recR* mutants are deficient at filling in daughter strand gaps in newly synthesised DNA [Kuzminov (1999) Microbiol Mol Biol Rev 63, pp. 751-813]. Mutations in *recR* delay induction of the SOS response in *E*. *coli* and such mutants are sensitive to UV-irradiation and mitomycin C [Keller (2001) Mutat Res 486, pp. 21-29].

In strain 26A10, the transposon has inserted in a gene with extensive homology to *thrC* of *H. influenzae.* The *thrC* gene encodes threonine synthase, the final enzyme in the biosynthetic pathway leading to the production of threonine (Patte (1996) Biosynthesis of threonine and lysine. In Escherichia coli and Salmonella (Vol. 1) (Neidhardt, F.C. et al., eds), pp. 408-433, ASM Press). Mutations in a *Streptococcus pneumoniae thrC* homologue attenuate the virulence of this organism in a murine model of pneumoniae [Lau (2001) Mol Microbiol 40, pp. 555-571].

In strain 26D12, the transposon has inactivated a GMP synthase, *guaA*, gene homologue. GMP synthase catalyses the synthesis of GMP from XMP as part of the purine biosynthetic pathway. Mutations in the *guaBA* operon attenuate the virulence of a variety of enteric pathogens including *E. coli, S. flexneri, and S. typhi,* and a *ΔguaBA* S. *typhi* derivative shows promise as a live attenuated vector [Wang (2001) Infect Immun 69, pp. 4734-4741; Noriega (1996) Infect Immun 64, 3055-3061; Russo (1996) Mol Microbiol 22, pp. 217-229]. Moreover, previous STM screens in *Streptococcus agalactiae* and *P. multocida* have implicated *guaA* and *guaB,* respectively, in growth *in vivo* and in pathogenesis [Jones (2000) Mol Microbiol 37, pp. 1444-1455; Fuller (2000) Microb Pathog 29, pp. 39-51].

Mutant 27A12 contains a transposon insertion in a gene previously identified in *A*. *pleuropneumoniae* as *tonB* (Tonpitak et al., Infect Immun 68: 1164-1170 2000) (now referred to as *tonB1).* This gene is found immediately upstream of, and is be co-transcribed with, *exbB, exbD,* and the transferrin binding protein *(tbp)* genes. In contrast, strain 0F6 contains a transposon insertion in an ORF encoding a protein with homology to TonB proteins from *P. multocida* and *Haemophilus sp.* (now designated *tonB2).* Moreover, sequence analysis of the region upstream from the transposon insertion in 0F6 revealed the presence of distinct *exbB* and *exbD* gene homologues, suggesting that A. *pleuropneumoniae,* in common with other bacterial pathogens including *V. cholerae* and *P. aeruginosa,* possesses two independent TonB systems (Occhino et al., Mol Microbiol 29:1493-1507 1998; Zhao and Poole, FEMS Microbiol Lett 184: 127-132 2000).

Strain 35D11 contains an insertion in a gene homologous to *H. influenzae dsbA.* DsbA is a small periplasmic protein and a member of the thioredoxin superfamily [Debarbieux (1999) Cell 99, pp.117-119]. Mutations in *dsbA* are pleiotropic, affecting the production of a variety of translocated proteins including bacterial virulence factors, components of type III secretion machinery, and c-type cytochromes including NAP [Yu and Kroll(1999) Microbes Infect 1, pp. 1221-1228]. Mutations in *dsbA* and *dsbB* have been implicated in the virulence of *Shigella flexneri* [Yu (1998) Infect Immun 66, 3909-3917] and *P. multocida* [Fuller (2000) Microb Pathog 29, pp. 25-38], respectively.

In strain 26C3, the transposon has inactivated a glutamyl-tRNA reductase, *hemA,* gene homologue while in strain 26D5 the transposon has inserted in a putative uroporphrinogen decarboxylase *uroD*/*hemE* gene homologue. Glutamyl-tRNA reductase catalyses the reduction of glutamate to glutamate 1-semialdehyde, the first committed step unique to the formation of δ-aminolevulinate (ALA) from glutamate. Downstream from ALA formation, uroporphrinogen decarboxylase catalyses the decarboxylation of uroporphrinogen III to yield coproporphrinogen, the first step in the conversion of uroporphrinogen III to protoheme via protoporphyrin IX. Hemes, including protoheme, are key components of the electron transfer apparatus and in addition play important roles as enzyme prosthetic groups in mineral nutrition and oxidative catalysis (Beale, Biosynthesis of hemes. In Escherichia coli and Salmonella (Vol. 1) (Neidhardt, F.C. et al., eds), pp. 408-433, ASM Press).

In strain 15A11, the transposon has inserted into a gene homologous to *P. multocida visC.* The protein encoded by *visC* encodes a putative monooxygenase belonging to the UbiH/CoQ6 family. UbiH is required for the biosynthesis of ubiquinone [Nakahigashi (1992) J. Bacteriol. 174, pp. 7352-7359], a prenylated benzoquinone that functions in the respiratory electron chain in the plasma membrane of prokaryotes. Ubiquinone and protoheme also function in the formation of disulfide bonds in periplasmic proteins by facilitating the oxidation of DsbA by DsbB [Kobayashi (1997) Proc Natl Acad Sci USA 94, pp. 11857-11862].

Strain 29A10 contains a transposon insertion in a gene homologous *to prfC* of H. *influenzae.* The *prfC* gene encodes peptide chain release factor 3 (RF3), a GTPase that in the presence of GTP catalyses the removal of RF1 and RF2 from the ribosome after translation termination [Freistroffer (1997) EMBO J 16 4126-4133]. The efficient recycling of RF1 and RF2 by RF3-GTP, although dispensable for viability in prokaryotes, is required for maximum growth rate [Kisselev (2000) Trends Biochem Sci 25, pp. 561-566].

In strains 26A6 and 33B7 the transposon has inserted in genes with homology to the *trmH* family of tRNA/rRNA methyltransferases. The interrupted ORF in strain 26A3 shows greatest homology to *H. influenzae yibK,* while that in strain 33B7 shows most similarity to *yjfH* of *H. influenzae.*

### (iii) Regulation

Virulence genes involved in environmental sensing and coordinate regulation were identified in this screen. The A. *pleuropneumoniae* homologue of the ferric uptake regulator, *fur,* was found to be inactivated in strain 6C12. Fur or Fur-like proteins have been shown to regulate virulence factor expression in a variety of pathogens including *E. coli* (Shiga-like toxin), *Corynebacterium diptheriae* (diptheria toxin), and *Pseudomonas aeruginosa* (exotoxin A) [Prince (1991) Mol. Microbiol 5 pp.2823-2831; Schmitt (1991) Infect Immun 59, pp. 1899-1904; Calderwood (1987) J. Bcateriol 169, pp. 4759-4764]. Strains 21D3 and 19B10 contain transposon insertions in the A. *pleuropneumoniae rpoE* and upstream *mclA (rseA)* gene homologues, respectively. The *rpoE* encoded sigma factor, σ^{E}/Sigma-24, is a member of the extracytoplasmic function subfamily of sigma factors that function as effector molecules in response to extracytoplasmic stimuli including heat shock and oxidative stress [Raina (1995) EMBO J 14, pp. 1043-1055; Rouviere (1995) EMBO J. 141032-1042; Ades (1999) Genes Dev 13, pp. 2449-2461; Dartigalongue (2001) J Biol Chem 276, pp. 20866 - 20875]. In S. *typhimurium,* σ^{E} is an important determinant of virulence and immunogenicity [Humphreys (1999) Infect Immun 67, pp. 1560-1568]. McIA (RseA) is an inner membrane protein that acts as a σ^{E}-specific anti-σ factor. MclA (RseA) is rapidly degraded in response to extracytoplasmic stress resulting in increased σ^{E} concentration and initiation of the stress response [Ades (1999) Genes Dev 13, pp. 2449-2461]. Finally, insertions in the A. *pleuropneumoniae* quorum sensing *luxS* homologue (strain 26D3) were observed to attenuate A. *pleuropneumoniae* virulence. Recently, the *E. coli luxS* homologue has been implicated in the control of intestinal colonisation factors encoded by the locus of enterocye effacement pathogenicity island in both EHEC and EPEC [Sperandio (2001) J Bacteriol 183, pp. 5187-5197; Sperandio (1999) Proc Natl Acad Sci USA 96, pp. 15196 -15201].

### (iv) Transport

The A. *pleuropneumoniae* gene *aopA* is inactivated in strain 23C9. The *aopA* gene encodes a 48 kDa immunogenic outer membrane protein that is common to all serotypes of A. *pleuropneumoniae* tested [Cruz (1996) Infect Immun 64 pp. 83-90]. Interestingly, AopA is highly homologous (75% identity, 84% similarity) to Nqr1 of *H. influenzae* (NqrA of *Vibrio alginolyticus),* encoding the alpha chain of the sodium-translocating NADH-ubiquinone oxidoreductase (NQR) complex [Hayashi (1996) FEBS Lett 381 pp. 174-176; Beattie (1994) FEBS Lett 356 pp. 333-338]. The NQR enzyme is a respiration-linked Na⁺ pump establishing an electrochemical gradient of sodium ions across the membrane. The resultant sodium motive force can be used for solute transport, ATP synthesis, and flagellar rotation. This alternative energy coupling of sodium ions rather than protons enables the bacteria to maintain a cytoplasmic pH near neutrality in an alkaline environment. The NQR enzyme has been most extensively studied in *V. alginolyticus* where it is thought to be involved in pH and ion homeostasis [Beattie (1994) FEBS Lett 356 pp. 333-338; Unemoto (1993) J. Bioenerg Biomembr 25 pp. 385-391]. In *Vibrio cholerae,* the NQR complex has been implicated in the regulation of virulence factor expression via its effects on ToxT expression [Hase (1999) Proc Natl Acad Sci USA 96 pp. 3183-3187].

Components of several different putative ABC transport systems were identified as necessary for virulence of A. *pleuropneumoniae.* ABC transport systems are involved in the transport (uptake or efflux) of a diverse array of macromolecules across the cytoplasmic membrane of bacteria and eukaryotes. These systems usually consist of three basic parts: one or two ATPases, one or two integral membrane proteins and a substrate-specific binding protein. Strains 13B12, 0C5. 19D1, and 24A4 contain transposon insertions in the ATPase component of ABC transporters. The gene inactivated in strain 0C5 encodes a protein with significant homology to MgIA of prokaryotic galactose transporters (Richarme et al., J Biol Chem 268: 9473-9437, 1993). The *in vivo* Cl of this mutant indicates limited availability of certain sugars within the respiratory tract environment. The interrupted ORFs in strains 19D1 and 24A4 are homologous to hypothetical ATPases from transport systems of unknown specificity in *P. multocida* (PM1728 and PM0996, respectively). In strain 20D10, the transposon has inactivated a gene with extensive homology to *znuA* of H. *ducreyi* and *pzp1* of *H. influenzae* (Lewis et al., Infect Immun 67: 5060-5068, 1999; Lu et al., J Biol Chem 272: 29033-29038 1997 and FEMS Microbiol Lett 165:129-1371998). ZnuA (PZP1) is a periplasmic zinc-binding protein that plays a key role in zinc uptake. Inactivation of the *znuA* gene in *H. ducreyi* caused a significant decrease in virulence in the rabbit model for experimental chancroid (Lewis et al.,Infect Immun 67: 5060-5068, 1999). In strain OD6 the transposon has inserted in a gene encoding a protein with homology to putative integral membrane proteins from *P. multocida* (PM0997) and *N. meningitidis* (NMB0549). In *P. multocida* PM0997 is located immediately upstream from the ATPase inactivated in strain 24A4 suggesting that they are part of the same transport system (May et al., Proc Natl Acad Sci U S A 98: 3460-3465 2001). This gene was also identified by Fuller *et al.* (Fuller et al., Microb Pathog 29: 39-512000) as *apvD,* the predicted product of which shows homology with the macrolide-specific ABC-type efflux protein MacA (Kobayashi et al., J Bacteriol 183: 5639-5644 2001). In mutant 9D10, the disrupted gene sequence encodes a protein with homology to CorC which mediates both influx and efflux of magnesium as well as cobalt efflux (Gibson et al., Mol Microbiol., 5: pgs 2753-2762,[1991]).

### (v) Stress

In strain 13A3, the transposon has inserted in a *dnaJ* gene homologue. DnaJ is an essential component of bacterial Hsp70 chaperone systems where it functions as a co-chaperone, providing substrate specificity to its Hsp70 partner, DnaK [Rudiger (2001) EMBO J 20 pp. 1042-1050]. In addition to their fundamental roles in protein folding and translocation, DnaK/DnaJ constitute the primary stress-sensing and transduction system of the E. *coli* heat shock response [Tomoyasu (1998) Mol Microbiol 30 pp.567-581] where they modulate the induction of the heat shock response by altering the stability of the heat shock sigma factor σ³². Significantly, mutations in DnaK have previously been shown to attenuate A, *pleuropneumoniae* virulence and to decrease the survival of *Brucella suis* in human macrophage-like cell lines [Fuller (2000) Microb Pathog 29 pp. 39-51; Kohler (1996) Mol Microbiol 20 pp. 701-712].

Strain 26B6 contains an insertion in a *htpG* gene homologue. HtpG is a heat shock-induced molecular chaperone that, in E. *coli,* contributes to the correct folding of cytoplasmic proteins in mildly stressed cells [Thomas (2000) Mol Microbiol 36 1360-1370]. HtpG does not appear to associate stably with partner proteins and little is known about its mechanism of action. It has been suggested that HtpG serves as a holder chaperone, transiently maintaining a subset of de novo synthesised proteins in a conformation that is accessible to DnaK/DnaJ [Freeman (1996) EMBO J 15 pp.2969-2979].

Strain 13D8 contains a transposon insertion in a *lon* gene homologue. Lon (also called protease La) is a heat shock-inducible, ATP-dependent serine protease that plays a major role in the elimination of abnormally folded proteins [Gottesman (1996) Annu Rev Genet 30 465-506; Suzuki (1997) Trend Biochem Sci 22 pp.118-123]. In addition to its demonstrated role as a stress response protease, Lon also functions as a pleiotropic regulator of gene expression by degrading unstable regulatory proteins including RcsA, a positive regulator of capsule synthesis [Keenleyside (1992) J Bacteriol 174 pp. 8-16] and SulA, an inhibitor of cell division and component of the E. *coli* SOS response [Mizusawa (1983) Proc Natl Acad Sci USA 80 pp. 358-362]. Recent studies in *Brucella abortus* have demonstrated that Lon is required for survival in murine macrophages and wild-type virulence in the mouse model of infection [Robertson (2000) Mol Microbiol 35: 577-588].

Strain 13C1 carries a transposon insertion in a *prc* gene homologue. Prc is a periplasmic protease whose natural substrates appear to be membrane proteins including TonB [Gottesman (1996) Annu Rev Genet 30 pp.465-506]. It has been speculated that Prc functions as a chaperone involved in the folding and turnover of proteins in the periplasmic space [Bass (1996) J Bacteriol 178 pp.1154-1161].

## Claims

1. An attenuated *Actinobacillus pleuropneumoniae* bacterium having a mutation in a gene required for bacterial virulence, wherein said gene comprises the nucleotide sequence SEQ ID NO: 10.

2. An attenuated *Actinobacillus pleuropneumoniae* bacterium having a mutation in a nucleic acid sequence responsible for, or involved in, controlling expression of a gene required for bacterial virulence, wherein said nucleic acid sequence comprises the nucleotide sequence SEQ ID NO: 47.

3. An attenuated *Actinobacillus pleuropneumoniae* bacterium according to Claim 1 or 2, having a plurality of mutations within said gene or within said nucleic acid sequence.

4. An attenuated *Actinobacillus pleuropneumoniae* bacterium according to any of Claims 1-3, further comprising one or more mutations within different genes or nucleic acid sequences.

5. An attenuated *Actinobacillus pleuropneumoniae* bacterium according to Claim 3, having a mutation in a gene required for bacterial virulence, wherein said gene comprises the nucleotide sequence SEQ ID NO: 10, and having a further mutation in a nucleic acid sequence responsible for, or involved in, controlling expression of a gene required for bacterial virulence, wherein said nucleic acid sequence comprises the nucleotide sequence SEQ ID NO: 47.

6. An attenuated bacterium containing a mutation in a nucleic acid sequence that hybridizes to a nucleotide sequence selected from SEQ ID NOs: 10 or 47, under conditions of moderate to high stringency.

7. An attenuated bacterium according to Claim 6, having a plurality of mutations within said nucleic acid sequence.

8. An attenuated *Actinobacillus pleuropneumoniae* bacterium according to Claim 6 or 7, further comprising one or more mutations within different genes or nucleic acid sequences.

9. A composition containing an attenuated *Actinobacillus pleuropneumoniae* bacterium according to any previous claim.

10. A composition according to Claim 9, comprising a plurality of different attenuated bacteria, wherein the attenuated bacteria have different mutations within said genes or within said nucleic acid sequences.

11. A composition according to Claim 9 or 10, further comprising one or more pharmacologically acceptable adjuvants, diluents, carriers, vehicles and/ or excipients.

12. A composition according to any of Claims 9-11, which is packaged in a form suitable for administration by intravenous, intradermal, intramuscular, intramammary, intraperitoneal and/ or subcutaneous injection, and/ or by oral, sublingual, nasal, anal or vaginal delivery.

13. A composition according to any of Claims 10-12, which is selected from the group consisting of an immunogenic composition, a therapeutic (e.g. prophylactic) composition and a vaccine.

14. A composition according to Claim 13, wherein the composition is a vaccine whose administration to an animal confers a degree of cross-protection.

15. Use of an attenuated *A. pleuropneumoniae* bacterium according to any of Claims 1-7 for the manufacture of a medicament for preventing or alleviating an infection of an animal with *A. pleuropneumoniae.*

16. Use of an attenuated *A. pleuropneumoniae* bacterium according to any of Claims 1-7 for the manufacture of a medicament for preventing or alleviating symptoms associated with A. *pleuropneumoniae* infection.

17. An isolated polynucleotide comprising a nucleotide sequence selected from the group consisting of:
(a) a nucleotide sequence selected from SEQ ID NO: 10 or 47;
(b) a nucleotide sequence encoding the polypeptide which is encoded by the nucleotide sequence recited in (a);
(c) a nucleotide sequence which hybridizes to the nucleotide sequence of (a) and/or (b), or to its complement, under conditions of moderate to high stringency; or
(d) a fragment of any one of the nucleotide sequences of (a)-(c), which fragment retains an immunological property and/or a biological activity of the recited nucleotide sequence of (a)-(c).

18. An isolated polynucleotide which is antisense to the nucleotide sequence of SEQ ID NO: 10, and which is capable of modulating the virulence of A. *pleuropneumoniae* by its direct interaction with an A. *pleuropneumoniae* virulence gene comprising the nucleotide sequence SEQ ID NO: 10.

19. An isolated polynucleotide which is antisense to the nucleotide sequence of SEQ ID NO: 47, wherein said antisense polynucleotide is capable of modulating the virulence of A. *pleuropneumoniae* by its direct interaction with an A. *pleuropneumoniae* nucleic acid sequence responsible for, or involved in, controlling expression of a gene required for A. *pleuropneumoniae* virulence, wherein said nucleic acid sequence comprises the nucleotide sequence SEQ ID NO: 47.

20. A vector comprising a polynucleotide according to any of Claims 17-19.

21. A host cell containing a polynucleotide according to any of Claims 17-19 or a vector according to Claim 20.

22. An isolated *A. pleuropneumoniae* virulence polypeptide encoded by a polynucleotide according to Claim 17.

23. A method of producing a virulence polypeptide according to Claim 22, comprising the steps of:
(i) culturing a host cell according to Claim 21, wherein the host cell expresses the polypeptide; and
(ii) recovering the expressed polypeptide from the host cell, or from its surrounding medium.

24. A composition comprising a polypeptide according to Claim 22.

25. A composition according to Claim 24, further comprising one or more pharmacologically acceptable adjuvants, diluents, carriers, vehicles and/ or excipients.

26. An antibody which specifically recognizes a polynucleotide according to any of Claims 17-19, or a polypeptide according to Claim 22.

27. A method for identifying an anti-bacterial agent that modulates the function of an A. *pleuropneumoniae* virulence gene comprising SEQ ID NO: 10, comprising screening potential agents for their ability to interfere with the expression and/ or biological activity in a host bacterium of the gene products encoded by SEQ ID NO: 10.

28. A method for identifying an anti-bacterial agent that modulates the function of an A. *pleuropneumoniae* nucleic acid sequence comprising the nucleotide sequence SEQ ID NO: 47, wherein said nucleic acid sequence is responsible for, or involved in, controlling expression of a gene required for bacterial virulence, said method comprising screening potential agents for their ability to interfere with the expression and/ or biological activity of said gene in a host bacterium.

29. An agent identified by a method according to Claim 27 or 28.

30. Use of an agent according to Claim 29 for the manufacture of a medicament for the treatment of a *Pasteurellaceae* infection and/ or porcine pleuropneumonia.

31. A method of modulating the transcription of an *A. pleuropneumoniae* virulence gene comprising the nucleotide sequence SEQ ID NO: 10, wherein said method comprises contacting said nucleotide sequence with a triplet helix-forming sequence specific oligonucleotide that hybridizes to said nucleotide sequence via oligonucleotide-directed triplet helix formation.

32. An attenuated *Actinobacillus pleuropneumoniae* bacterium.

33. Use of an attenuated *A. pleuropneumoniae* bacterium according to Claim 32 for the manufacture of a medicament for:
(i) preventing or alleviating an infection of an animal with A. *pleuropneumoniae;* or
(ii) for preventing or alleviating symptoms associated with A. *pleuropneumoniae* infection, such as the prophylactic protection of swine against porcine pleuropneumoniae; or
(iii) for the therapeutic treatment or prophylactic protection of an animal against infection by the corresponding wild-type bacterium, or a different strain or serotype thereof.

34. An isolated polynucleotide encoding a gene product which is naturally involved in (e.g. required for) the virulence of A. *pleuropneumoniae.*

35. An isolated polynucleotide encoding a gene product which is not naturally found in *A. pleuropneumoniae,* but whose expression therein is capable of modulating (e.g. of decreasing) the virulence of that bacterium.

36. An isolated polynucleotide which is not naturally found in *A*. *pleuropneumoniae* but which is capable of modulating the virulence of that bacterium by its direct interaction with A. *pleuropneumoniae* virulence genes or gene products.

37. An isolated polynucleotide comprising a nucleotide sequence selected from the group consisting of:
(a) a nucleotide sequence selected from the group consisting of SEQ ID NOs: 1-56;
(b) a nucleotide sequence encoding the polypeptide which is encoded by the nucleotide sequence recited in (a);
(c) a nucleotide sequence which hybridizes to the nucleotide sequence of (a) and/or (b), or to its complement, under conditions of moderate to high stringency; or
(d) a fragment of any one of the nucleotide sequences of (a)-(c), which fragment retains an immunological property and/or a biological activity of the recited nucleotide sequence of (a)-(c).

38. A vector comprising a polynucleotide according to any of Claims 34-37.

39. A host cell containing a polynucleotide according to any of Claims 34-37 or a vector according to Claim 38.

40. An isolated A. *pleuropneumoniae* virulence polypeptide.

41. A method of producing a virulence polypeptide according to Claim 40, comprising the steps of:
(i) culturing a host cell according to Claim 39 under conditions that permit the expression of the polypeptide; and
(ii) recovering and optionally isolating the expressed polypeptide from the host cell, or from its surrounding medium.

42. An antibody which specifically recognizes a polynucleotide according to any of Claims 34-37, or a polypeptide according to Claim 40.

43. A method for identifying an anti-bacterial agent which is capable of modulating the function of an *A. pleuropneumoniae* virulence gene of the present invention, or of a homologous gene in a related species.

44. An agent identified by a method according to Claim 43.

45. Use of an agent according to Claim 44 for the manufacture of a medicament for the treatment of a *Pasteurellaceae* infection and/ or porcine pleuropneumonia.

46. A composition containing a bacterium according to Claim 32, a polypeptide according to Claim 40 or an anti-bacterial agent according to Claim 44.

47. A method of modulating the transcription of an A. *pleuropneumoniae* virulence gene through the use of oligonucleotide-directed triplet helix formation.


SEQUENCE LISTING

<110> Imperial College Innovations, Ltd.

<120> Bacterial Virulence Genes

<130> MRM/PJG/24342

<150> GB 0228691.2
<151> 2002-12-09

<160> 63

<170> PatentIn version 3.2

<210> 1
<211> 3344
<212> DNA
<213> Actinobacillus pleuropneumoniae

<400> 1
gaattctaaa ttgagctgcc aatgcttgtt gttcgtcaca attcacggtt gccaataaaa 60

attgtgccgg attttcatcc gccaaccggc gtaataatga atccatttcc aatgaagcca 120

cttctctcgg cgagataaaa ttaaacacga ccggcacttg tgtcgccgca ttccacactt 180

cactaaaatt actttcggta acattgacaa aataattcat actcttttcc taatctaaaa 240

aatcggcaaa ttatacttta tttttagtaa gccgtttcaa tcggtaaacc gcttctttcc 300

caaccgtcga aaccgccttt cacgctatat acgcgatcaa aaccctgttc gactaaaaat 360

tgtgcggtat tacgactgct cacgccgtga tagcacatga cgatgaccgg ttcttcataa 420

tccacttcat ctaaaaaacg cccgtagctt tcattagtta aatggaacgc atcttgcgga 480

tggctatagg cataacgacg tccatcacga atatccgcta aagtcgcacc ttcgttttcg 540

attaattccc acgcctgatg cgggctgatc tcagtaaatg tttcactcat atttcatgct 600

caataagaaa aataaaccaa ataaaattaa tcgactatac cataagacgt ttacaaaaat 660

aaacgagaat acctattatt acgagttgaa ttttctgact tacttagtat aattgcaaac 720

cattttcatt atacagaact cactgtttgc ttcttttaga ctgtttccga ggattttatg 780

gaacaaatgc ttgaactttt acaaggtcat gttgattata ttattttagg cttattacta 840

ttaatgagtg ttgtgttggt atggaaaatt attgaacgcg tacttttcta caaacaattg 900

gatgtgacca aatatgacac gctacaagat ttggaaattg ataccactcg caatttaacc 960

accatttcca ctatcggtgc caacgcccct tatatcggtt tattaggaac cgtattaggg 1020

atcttactta ccttctatca tttagggcat tccggcggtg atattgacgc cgcatccatt 1080

atggttcacc tttcgcttgc attaaaagca accgcagccg gtatcttagt cgctattccg 1140

gcaatgatgt tctacagcgg ttttaaccgt aaagtggatg aaagcaaact taaatggcaa 1200

gcgattcaag ctcgtaaagc caatcaataa gcggtcaaat ttcacttatt ttttgcaact 1260

caatcaggct tcttatgaaa aaatttgacg aaattaatat tattcccttt atcgacatta 1320

tgttggtatt actggctatc gtgttggtaa ccgcttcatt tatttctcaa ggaaaaattc 1380

cggttaatat tccgaaagcg accacaactc aagcgatgaa agcggacgaa ttggcaaaac 1440

ttctgacgat taccgaaaac aacgagtttt atttcaacga tcagccgatt accaaagaag 1500

cactcacgag cgagattgca acttgggata aaacccaaaa agtaacctta aaagtggacg 1560

gtgcggtagc ctttgaaaaa tttgtcgaac tgactgatgt tctctctgcg aatgaaatta 1620

agaatgtcgc tatcgtgacc aaaaaagata cctcgaagaa ataggatacg agtatgaaga 1680

aaaaacattc tcgtatcggt ttaaccggct ccattcttat ccataccgct attttcggcg 1740

gtttagcctc tatggtaacc cattctcatt ccgatgattt accccaagaa gaaacgatgt 1800

ctatggagtt agtcgccgca cttttggaac aacctcaagt tgccgtcgca caagaagagc 1860

cggcaccgcc ggagccggag aaagtcgaag tcgagccgga accggagcct gaagcggaag 1920

ctattccaga acttaaacct cagcctaagc cgaaagaaaa accgaaggaa aagcctaagg 1980

aaaaaccaaa accgaaagaa ccgccaaagc ctaaagaaaa agaaaaacct aaaaaagaga 2040

aagtaaaaga taaaccgatt aaggcgttag aaaaaggtcc tgaagcaaaa caaggtattg 2100

tcgccaaagc gattcctaat gcggtacaag gcactcaatt acgtgcaggt attccaaacg 2160

gtaaaccgga aggtaatccg aacggtcttt ccgccaacgg ttcacaaaac ggtgcggtag 2220

gcggtagtgg tagcggagct tccggaagtg aaatcggtgc gtataaagcg gcattacaac 2280

gtgcattaca acgccgagcc aataacgctt atccgactcg agaaaaaatg atgcgcaaaa 2340

cgggcgtggt tactatcggt tttaccgtat cctcttccgg cgagttaatc aatgtcaaag 2400

tgttaaacag ttccggtaac ggtaatcttg ataatgcggc ggtcaaagcg gcacaaagca 2460

cgaaagtttc accgccgccg gcgggattcc cgagtaatgt gaccgttccg gtgaaattct 2520

cgattgaata aatcttgtca ataagctccc gtaagggagc ttatttttac ttaagccaag 2580

cggtcgtttt tagcttactt gttgcaaaaa atcgacaaaa gataaccgct tgcctaaagc 2640

ggagtgctta agtaaaataa accgtttcta tttttattat acaaattgag gaaaatataa 2700

tgtttggaaa aggcggctta ggcggtttaa tgaaacaagc tcagcaaatg caagagcgta 2760

tgcagaaaat gcaagaagaa atcgcacagc ttgaagtcac cggcgaatcc ggtgcgggtt 2820

tagtcaaagt aacgatcaat ggcgcacata attgccgtcg tatcgaaatc gatccgtcat 2880

taatggaaga cgataaagag atggtggaag atttagtcgc tgccgcattc aatgatgccg 2940

ttcgccgtgc ggaagaaatg caaaaagaaa aaatggcaag cgtgaccgcc ggcatgcaat 3000

taccgccggg catgaaattc ccattctaat ttagagataa tggatagttc gctttattgc 3060

ggctatccat tattaatttt caactatccg gtctattaaa gcaaattagc cctttactcg 3120

aaaatctgat ggaagacata agagccctac gtgcattacc cggcgtcggt ccgaaatccg 3180

cacaacgtat ggcgtatcat ttattgcaac gcaatcgttc cggcggtatc aatttatcca 3240

aagccttaaa tgaagcgatg acgcatatcg gtcattgccg ttcttgccgt acttttaccg 3300

aagaagacga atgcaatatt gttgatgcgc tggcagtgtt cctg 3344

<210> 2
<211> 1080
<212> DNA
<213> Actinobacillus pleuropneumoniae

<400> 2
ttgaaagttt caccatatat tggctcaagt tattattcat ctaaatatac agattattac 60

tttggtatta aacaatccga attaggtaat aaaattacat ccgtatataa acctaaagca 120

gcttatgcaa cacacatagg tattaatact gattatgctt tcacgaacaa tcttggcatg 180

ggtttatctg tcggttggaa taaatattct aaagaaatta agcaatctcc tatcataaaa 240

cgagactctc aatttacttc atctcttagc ctttattata agttctaaaa tagaatattc 300

tagggagaat actcattctt tatctttata aagttaattg tttctccctg tttctatatt 360

atttagttac ttgttcaaaa gctacattgg ttattttgtc attttataaa agataataag 420

gtggttattt tgaaaattaa gaaatatatt aaatataccc tatttacttt ccttttaggc 480

atatcatatt tatattttgg gggcgaaaac gaaaattatc aagagattat ttttcacgtc 540

gaacgaggaa atattcaaaa aacagttatt gctagcggca cattgcaagc gactgaacaa 600

gtagatattg gtgcacaagt atctgggcag attaagcata ttttagtaca agaaggacag 660

aaggttaaaa aaggtgagct attagctgta attgatccac gtctggctga aacggaatta 720

aaactagcaa aagctgagct agcaaatgct tctgctaatt tggatacaaa aaaaattaat 780

cttaagcaac tgcaatcaga ttgggaacgt catcaacgtt tgatacgaac caatgcgaca 840

agccaaaagg aaacagaaga agcaaaaagt agattaaata cggccaaagc agaacttcaa 900

attgcgcaaa ataatctaga tatcgctaaa atcagagtgg aaaaagctga aaccgaacta 960

ggatatacag aaattcgttc tccacttgat gcaacagtaa tttcagtatt tgcgcaaaat 1020

ggtcaaactt tagtcaccac ccaacaagta ccagtgctga tgaaattagc taatattgat 1080

<210> 3
<211> 582
<212> DNA
<213> Actinobacillus pleuropneumoniae

<400> 3
cgggtattat ttatatttcg cacaaaatgg atgaaatctt caaaatttgt gacgaaatta 60

caatcttacg tgacggtaaa tgggtcaata cggttgcgat taaagacacc tcaatggaac 120

aaatcgtttc gatgatggta ggtcgtgaac tgacccaacg tttcccggaa aaaaccaata 180

agccgaaaga aaccgtactc gtggtcgaac atttaaccgc taaaaatcag ccgtctattc 240

aagatgttag ctttgaattg cgtaaaggcg aagtgttggg tatcgccggc ttggtcggtg 300

caaaacgtac cgatattgtc gaaacgattt tcggtgtgcg tgaaaaagcc ggcggaacta 360

ttaccttaaa cggcaaagcg gtaaacaatc gtaatgcgtt tgaagcaatc aataacggtt 420

ttgcgctggt tactgaagaa cgccgctcaa ccggtattta tgcgaatctc agcatcgaat 480

tccactcgtt gatttcaaat atgaaatctt atttaggtaa gttcggctta ctcagcaata 540

aaaaaatgaa aagcgatacc cagtgctgat gaatccccta at 582

<210> 4
<211> 1264
<212> DNA
<213> Actinobacillus pleuropneumoniae

<400> 4
ggactttcta aacacgaacg gccaaaagcg ggttgttggt ccggtatcgt cgggatggcg 60

attgcgttag tcgcaactat cttcgggtcc gcaatcacaa ggtacgcttt ggatttaatc 120

gcaatggcaa tcggcggatt tatcggtgtg agaaaagccg taaaagttga aatgacggaa 180

atgccggaac tagtggcgat tctacatagc tttgtcggtt tagctgcggt attagtcggt 240

tttaacagct acggtttaca cgtagatgcg attccaccgg caaacttaga tgaagtggca 300

ctagcggcat tccaagccga acaagcgata ctcgctaaca ttcataatgt tgaagtgttc 360

ctcggtatct ttatcggtgc ggtaacattc tccggttcat tagtggcgtt cggtaaatta 420

agcggtaaat tattcggtcg taaagtgtct tcggctgcat taaatattcc gcataaacat 480

aaatggaact tagcggcatt aatcgtttct gcgttattaa tggtggtatt cctaaataat 540

ccgcacaata ttttccctgt gttgattatg acggtaatcg cattagtatt cggttggcac 600

ttagtatcct ctatcgctgg tgcggatatg ccggtagtgg tttcaatgtt gaactcatat 660

tcaggttggg cggcagcggc agccggattc atcagtgcta acaacgactt attaattgtg 720

actggtgcgt taggggttca tcagcgcgat tctttcttac atcatgggga aagcgggaac 780

cgctcattta tcagcgtgat tgcaggcggc ttcggtacgg aagttaaagc aagctcaggt 840

gatgaagaac aaggtgaaca ccgtgaaacc accgcagaag aagtggcgga aatgctgaaa 900

aatgcaagtt ctgtaatcat cactccggga tacggtatgg cggtggcgca agcacaatat 960

ccggttgcgg aaatcactgc aaaattacgc gagaaaggtg ttaatgtgcg ttttggtatc 1020

caccctgttg cgggtcgttt accggggcat atgaacgtat tacttgcaga ggcaaaagtg 1080

ccttatgatg tcgtgcttga aatggatgaa atcaatgatg atttcgaaga aaccgatgtc 1140

gtattggtta tcggtgcgaa tgatacggta aaccccgcgg cattagacga tccgtcaagt 1200

ccaattgcag gtatcccgta tttgggaagt gtggaaagca caaaaccgtt atcgtatcaa 1260

cctt 1264

<210> 5
<211> 574
<212> DNA
<213> Actinobacillus pleuropneumoniae

<400> 5
tacgcaacag gataaaaaat cgtttttaca atcgattttc agcggcttat ttcaatccga 60

accgaaaaat agagaagact tggttgaagt aattcgagat tctctggata acgaactgat 120

cgatagtgat accaaagata tgatcgaagg cgtaatggag atttccgaac tgcgtgtgcg 180

ggatattatg atacctcgtc cgcaaatcgt ttatattgac gccaatcttg atcttaccgc 240

ttgtgttgat ttaatcattg aatccgctca ctctcgcttt ccggtcattt tagacgacgg 300

caaagatacc gtattaggta ttttacatgc caaagatctc ttaaaattcc tcagaaccga 360

ttccgaagag tttgaaatgc caaccattct acgccctgca gtgattgtgc cggaaagtaa 420

acgggtcgat agaatgttga aagaattccg ctccgaacgc tttcatatgg cgctggtagt 480

ggacgaattc ggtgcggtat ccggcttagt cacgattgaa gatattctcg agcaaatcgt 540

cggcgatatt gaagacgaat tcgacgaaga agaa 574

<210> 6
<211> 575
<212> DNA
<213> Actinobacillus pleuropneumoniae

<400> 6
gatatcgtat cacataagta aagaccgctc gcataagcga agcggttcta atttcatcta 60

aagttttact cgtgtactga cgaagtaatg caaggatttg catcagtttt ctcttgcatt 120

aatactttat cgccgcgagt ttctaaattt aagcgtacaa caggttcggt atttgaactg 180

cgtaagttga aacgccagtt ttcaaattca acgctgatac cgtctaaggt ttcgacttta 240

ccgtctttat acgcttcttt aacacgttcg atagccgctt tagcatcagt taatttactg 300

ttaatttcac ccggtgacgg gaatttatcc aaacgagcac cgactaattc gcttaacggt 360

ttacctgtcg tacatagtag ttctaacgtt aatagccatg gaatcatacc gctatcacag 420

tagaagaaat cacggaaata atggtgagca gacatttcac caccataaat cgcatctact 480

gcgcgcattt tctctttaat aaacgagtga cccgattttg acatcaccgc ttcgccgccg 540

gcttccgata ctaattcttc cgtattccaa attaa 575

<210> 7
<211> 379
<212> DNA
<213> Actinobacillus pleuropneumoniae

<400> 7
tactaaacct tctttaccac ctaagataga aacgaacgca ccgaagtcca caacgcgagt 60

tactttacct ttgtagatcg cgttcacttc aacttcagcc acgatatctt cgatacgagc 120

cattaccgct ttcgccgcat tattatcggt tgcggcgatt tttacggtac cgtcgtcatc 180

aatatcaatc gaagtaccgg tttcttcggt taatgcgcga attaccgcac cgcctttacc 240

gatcacgtct ttgattttct tcggatcgat cttcatcgta tgaatacgag gcgcaaaatc 300

ggaaatatcg gcacgaggtg caggaatcgc ttgttccatc acgcttaaga tgtgcatacg 360

cgcacctttc gcttgattt 379

<210> 8
<211> 876
<212> DNA
<213> Actinobacillus pleuropneumoniae

<400> 8
attagcagta acagggtgat taacttataa tttatagggg tttttatgaa atcagataaa 60

ccggttgaat gtgtgggttg taacactttt gatgtcggct caatcattga taatagtgaa 120

cgagatgcga aatttgcaaa agtatatgaa acacaagcgg aagcggaaca agcacttgtg 180

aaattaaccc aaaaagcacg cgatacggaa agcgaaccgt gtgaaatcac cagtgaaatt 240

aaagcggtag agggcggttt tttactggat gcgaatttcc gttttagttg ccaagcggaa 300

gtggtaattt tccaattagg tacacgcgca atccgcgcaa tctaatttta ttaatttgaa 360

aagcggttgg attttgctga aaatttgcaa aaaatgagca aaatccaacc gcttttgttt 420

tcggagaaat tgatgaatcc ttgggtttta ctaggctttg cgattgttat tgaagtgatt 480

ggcagtaatt gcattaaggc aagcgaaggt tttagcaaac cgctaccgac cgtagtggcg 540

atcggcgctt ttgtgatagc gttatattta ttgtcggtga ttaccaaaac attgccgatt 600

ggtattgttt atgctgtgtg gtcgggtgtc ggtatcgtac tgaccgccat tgtggcatat 660

ttcgctttcg ggcaaaaacc ggatttggcg ggctttatcg gtatgggaat gattattgcc 720

ggcgtgctag taattaactt attctcaaaa gcatctactt aattttgaaa tgtgatacaa 780

gcggttgatt ttcacaagaa ttttgcaaat ttttcttcaa aatcaaccgc ttgtttttgc 840

ttaaggctgg tcgccggcta acgcacgagg gaataa 876

<210> 9
<211> 1251
<212> DNA
<213> Actinobacillus pleuropneumoniae

<220>
<221> misc_feature
<222> (489)..(489)
<223> n is a, c, g, or t

<400> 9
agtcatcggt atcttcggtt gcggtgacca agaagattat gcggaatatt tctgtgatgc 60

gatgggaacc attcgcgatg tcgttgagca aaacggcggt gtgattgtcg gacattggcc 120

gacggaaggt tatagctttg aagtatcgca agcgttagtg gacgaaaata catttgtcgg 180

tttatgtatt gatgaagatc gccaaccgga attaaccggt gaacgtgtaa accgttggtg 240

taaacagatc tttgatgaga tgtgcttaga tcagttagca taatgaatat aagagagaga 300

agggtaccaa gttatgtctg aagaaaatgc aaaacttctt aaaagtgtag gtttgaaagt 360

tactgagcct cgtttgacca tccttgcgtt aatgcagcaa catcgcgaac aaatgctaca 420

tttttctgcc gaggatattt ataaaatttt attagagcag ggttccgata tcggtttggc 480

aacggtttnc cgcgtattaa accaatttga agaggtcggt attttacttc gccataactt 540

tgatgcgaat aaagcggtat tcgagcttaa cgtagagcaa gaacacgatc atattatctg 600

tatggattgc ggtaaggtat tcgagtttaa agatccggat atcgaacgcc gtcagcgtga 660

aatcagcgaa cagcacggta tgaaattagc gacgcacagt ttatatctct atgcgaaatg 720

tagcgatatt tcacattgtg attcttcaaa agataaaaaa taatttatct tatcgttcag 780

tataaagcag acatataccg aaggttttgc aggccttcgg ttttgtgtta tctaaaggat 840

atgaaattaa gcggttgttt tgtaaaattt ttagtaaaaa cgaccgctat ctgaaagaga 900

cctcgattta atgacggata atatttcaca gcctgtagag gccaaggttc gacagcctcg 960

taaaatctca cctttttggt tactaccaat cgttgctttc gtgattggcg gattgttatt 1020

tttccagatc cttaaagagc agggcgaaat gatcacgatt cgtttcaacg aaggcgacgg 1080

gatcaccgca ggaaaaaccg tcattcgcta ccaaggtttg cagatagggc aagtgaaaaa 1140

agtctatttt gtagaagatc ttaaaaaagt cgaagtgcaa gcggaagtga atcccgaagc 1200

caaatccgtg ttacgagaac aaaccaaatt ctggtggtaa aaccgagtcc t 1251

<210> 10
<211> 748
<212> DNA
<213> Actinobacillus pleuropneumoniae

<400> 10
gattcgattc aatcggtaga aatgccggcg tctatggcat cggctccgtc acggacaatc 60

gagcaaaata ccgcttataa taatcaaacc gcttatacgc aaccggttaa tagcgtaccg 120

caacagcctg tggttcaaac tccggtacaa tccgcaccga gctatacaag cgaaagcggt 180

gccggtagca atatgattgg taattgccga gtggtaagag atgcgttagg tgcaccggtt 240

tatgcggaaa ttaccaaagg ttgttatacc gatagtagtt atacggtcgg aaaaagcgat 300

acaatgtatt taatttcgta tcttaccggt caaacgccgg cacaaattgc ggcattaaat 360

aatctcagtg tagagagtaa attacacgta ggtcaaatac tgcgtgtaag ataacggtag 420

gtttattttg ttagtattaa cgacagttgt gctttgccgc tgtcgtttta ttcgttttta 480

gattttaaag gaaagtaata tgaaaaaggc atttttatta ttgccgttta tggtaagcct 540

gaccgcttgc ggagcaagtg agaacgcacc gaaagacgat gcggaattgg cgccggggat 600

tatgcaaccc gtttccggat caggcgcatc gacaggaagc tacggttggt cgtcagacgt 660

tgagtccgca ccgatgccgg cttcaatgac taaataatcg ttttagcata cgaaaggata 720

aattgaacag atgaagaaat catttttt 748

<210> 11
<211> 511
<212> DNA
<213> Actinobacillus pleuropneumoniae

<400> 11
tgcaacgctt gttacaagaa ggctggcaat tacagccaaa agcgttatcg gacggcccgg 60

cgaaagtgct ttcacaccct gattaccaag gcgaaatcgg tttgattatg ccgtatgaga 120

aaaacttctg cgccagctgc aaccgcctgc gtgtttcggc actcggcaaa ttgcaccttt 180

gcttattcgg cgaggaaggc attgatttgc gagatttact ctcggaagat acccaacaag 240

cgcaattgaa agcacgctta aaagccgcat tacaaggtaa acgggaacat cactatttgc 300

atatcggtga tagcgggatt cgaaataacc tcgcctcaat cggcggctag tttctctgtt 360

acttttcttt gcgttgccaa agaaaagtaa ccaaaagaaa ggcaacccta cttcaccgct 420

gttcttcact tagttgaaat ttgcttaacg gaacatttta gattcgccta cggctcaaga 480

aaaatgttcc tacaaattgc aacacgctca g 511

<210> 12
<211> 1218
<212> DNA
<213> Actinobacillus pleuropneumoniae

<400> 12
tgctaagcat gaagaacgta ttgcgaaaga acctgagttt attacgttaa acgaagatat 60

tgcaatccgt aaagaaagag gcgctcgcaa gtattcgtca ctgaatctgg aagagcgttt 120

gaaagaagat cgtgccgacg aagcgaaacg tctgaaagat ctgaatgcac gctttgcaag 180

ggaagggaaa aaagcgatta agaatattga cgccttaccg aaagactatg aagcgccgga 240

ctttttctta aaagaagctg aaaaaatgat ggttgattgg ttagaaattg ataagaagtc 300

ataaaatagc ttataatgta ttaaattagc tgccgcttaa tgaaagttag gcggtttgtt 360

tttattgata ttttataatc tcggagctaa ggatttttat atgagtaagg cgaaaacctt 420

caaattactg ccgttagtaa tgctttcaag tgtcgtttta gccgaaacta atgcaactca 480

tcttcaggct gcatcaacag aaaacgcaac ggttaatcat gcgacatcgg atgcaatttt 540

accccagcta agttttagcg ctcagcaggc tagagcggct gaacttgcaa aacgtgcgga 600

ttatgttttt gagcaagagc agattcgttt ggaagaagaa cgtaaattgc aagaagcggt 660

gttgaaagta aagcaaacgg tcggagataa tcagttatta ctaactaaat cggttgctaa 720

aatttatttg gataacgatt atgcaagtat gtggaatgac aagcatgcgg agaagcaatt 780

cctcaaggaa tacgcattat ttgccgcaag cggcgtatca agcaaatcgg ccaaagcatt 840

acaacagatt ttaaatcatc cggaaggttt gggcagagat atcttattaa ccgacggttt 900

cttagattat ctctattaca ataaaaacgt gtataaaaat gcgaatcaat ggttatacaa 960

ccttggcaac cttggcagct atagccctaa atcacctagc tcggaacaaa taaatcaatg 1020

ggcggaatcg actaagaacg gtgattcggc tcacttcggg gcaaatttag tgcctcgtaa 1080

tcatatttat caagaaacgg ggcaacgcgt cttagcgatg tctgccggcg ggaatacgga 1140

gaaagtagca aaagcaaaga aaacaaaaac gcctgagctg gcaacggcaa ccggtaacga 1200

tacgttctat aaattagc 1218

<210> 13
<211> 712
<212> DNA
<213> Actinobacillus pleuropneumoniae

<400> 13
attctaggtc gtcagtctga gaatttaact cgtgcatggg catatgcgat ggcgttaggt 60

gtggaagata aagtaaaaac cgcactcttt gaagcggcgc aaaaagatgc aatgaaatca 120

atggacgata ttaaagcggt atttactgca aacggcgtaa gtgcagcgga ttttgataat 180

ggcattaata gctttgcggt aaatggtttg gtaaacaaac aagtacaatt agcagaagat 240

tttaaaattc gcggcgtacc ggcgtttttc ataaacggac agtaccaaat caatttggaa 300

ggctttgccg attccgcatc aaccaacgaa tttgtgaaac gttatgtaga agcggtgact 360

tttttaatta aaaagtaaat taagggcata gtagacaaaa tggttgctat tttttgaaaa 420

actatgccaa gtggacaaag tggtggctaa atagtatttt ttattttatt tagccactct 480

ttttatttaa aaaatccttt ataaacataa cattatgtga cttacttaat ccattatgat 540

tatttaattt ccgcttcagt tcactaaata atccttccag tctatttgtc gtcttttcaa 600

tatttaattc cgggtatttt tcataagtaa aaatataatc catataacgt ttaatactcg 660

cataagcact tctcacattt caatgtttat agggaaaata accctgctca tt 712

<210> 14
<211> 496
<212> DNA
<213> Actinobacillus pleuropneumoniae

<400> 14
tgcttagttt agacggtgta ccggcaatta aagcgattgt cgatgcaacg gaaaaacatg 60

ataacgatga aaagcacgag catgatcacg accataagga cgatcataaa catgaccacg 120

acgatcataa acacgaccat gcagggcatg atcatcacca tgatcacgaa gggcatagcc 180

atgataaaga ttggcatatt tggttctcac cggaagcaag ccttgccgcg gcggaacaaa 240

tcgcagcacg tctaagcgca caattaccgg aacaaaaagc gaaaattgcg gaaaaccttg 300

caacatttaa agcaaattta accgctaaaa acgaacaaat tcgcaatcaa ttagcagcag 360

taaaaggtaa aggttattat actttccatg atgcttacgg ctatttcgaa cgtgcttacg 420

gattaacctc attagggtcg tttacgatta acccgagcgt agcgccgggg gcgaaaacgt 480

taagcgtaat taagaa 496

<210> 15
<211> 408
<212> DNA
<213> Actinobacillus pleuropneumoniae

<400> 15
agctctacat catctaaata accgaattca gcagcaaaaa gaactaatgc ttgttcgctt 60

accagcattg gagcaaattg tttttgttta agtaattccg tcactttttg accgtgagaa 120

agctgtttac gagtcgcatc atctaaatcg gaagcaaatt gagcaaacgc cgctaattca 180

cgatactgag ctaatgcggt acggataccg ccagataatt tcttgatagc tttagtttgc 240

gccgcagaac ctacacgaga tacggaaata cccgggttta ccgcaggacg aatacctgag 300

ttaaataaac ttgactctaa gaaaatctga ccgtcagtaa tagaaattac gtttgtcgga 360

acgaaagccg atacgtcacc cgcttgtgtc tcgataatcg gaatgcag 408

<210> 16
<211> 476
<212> DNA
<213> Actinobacillus pleuropneumoniae

<400> 16
cgcttaaccc cgttactaaa tgagttcaac gtttaggtat ttccgttcag caagtaaacc 60

gccaaacatt ggataacaaa gcgcaaggtg aagttcacca agggattatt gcgaaagtcg 120

tgccgcaaaa agagctgaac gaacatgatc tggatgctat tctggcgcaa aaacaaaacc 180

cgtttttatt aatcttagac ggtgtgaccg atccgcataa ccttggtgct tgtttgcgta 240

ctgcagatgc ggcaggtgtg gatgcggtga ttgtaccgaa agataaatcg gcacagctta 300

cctcaaccgc tcgtaaagta gcttgcggtg cggcggaaac cgtgccgtta attcgtgtta 360

ccaaccttgc tcgcacaatg cgagagctac aagagcaaca taatatctgg attgtcggca 420

cggcaggtga agcgacttcc ggtatttatc aggctaaatt aaccggtgct atcgct 476

<210> 17
<211> 1011
<212> DNA
<213> Actinobacillus pleuropneumoniae

<400> 17
attgcgcaaa atgctaacag aattatcaaa atcaaagatg gcgaaattat tgcagataat 60

agttcatact gcaagaatag cctctccccc tcaacaaagc aggaaaggca aagtctaaac 120

tgcttgatgt cgctacgagg ctataaagaa tcttttgtga tggcattcag tatgatgcgt 180

gctcataaaa ttaggacgtt tttgactatg ttaggtataa ttattggcat tgctgctgtc 240

gtatgtgtag tagcattagg agaaggaacc aaacataaag tcttgaatga atttagctct 300

ttaggtagca acacaataga tatttttcct ggtaaaaatt ggggagatac cgaagcatat 360

aaaattcaaa cgttaaatag caatgacgta aatttattac gccaacaacc ctatgtgaaa 420

ggtgcgactc cgaacttatc gttagaattg ccaatccgtt tcttaaataa aactgccaat 480

gcaacgataa atggtgttag ccaagatttc tttatgttaa aaaactacaa attactaagt 540

ggcagatttt ttaaccaaca agatgtagat acttatcaag ctgttggcat aattgataaa 600

aaatcacgag atgtaatctt cggcacaaaa attgcagaaa gcaatatagt ttttatcggt 660

gatgtaccta tttcgattat cggtgttgtt gagtccgcct cccaaacaac tgaagggcag 720

cgaaccacta tttggttacc gtacaacact atggctgctc gtttacgtaa ccaaccctat 780

tttcaacaaa ttacagtgca gttacgagaa aattttgtac cagcagcagc cgataaggct 840

attgtggatt tattggcagc aaagcaatcc atatcatggc agataggatt tttttacttt 900

cagtagcagc aagttcttgc aatccttaaa tcgcactaca cagccctaac tttaatgatt 960

tcatctatcg cttttatttc gctggtagtt ggaggtatag gagttatgaa t 1011

<210> 18
<211> 650
<212> DNA
<213> Actinobacillus pleuropneumoniae

<400> 18
tcgacgctga gcgtctttat atgggcaatg gtgaggggta gttttacctt ttgcataggg 60

tgtgcatatt acagatagaa agcaaaatag tcaaagagaa aatcggattt ttaaaaaata 120

tctcataagc taaactaaat aaaccgaacc ggtttgatat aaggtaaact tatagaaatt 180

tttcacgtct aaccatagca aaaaagcgat aaaactggta atttgactat tcgtatctga 240

aaactattct tatttttaat ttaaacggga tgacaatgtt atttgcgatt ttagcaatgc 300

tggcttatgc tgccgcatta ttatgggtta cccccacact cgtcaattta gaaaaccaaa 360

ccggtgataa aaaaccgaat cttaaagcgg tttttggatt cggcttactt gccgtattct 420

ttcacgcaat caatttatcg caagaattgg tgctgagcgt gctgagcgag ggcggtcaga 480

atttctcact ttctaacgta aactcaatga tgagcttatt gctgacggct gtcgcaacac 540

ttgccttacc gcgttggaaa accatttggt ttccgttaat ggtggtttat accttcggca 600

tttgtagcgt agcggcatcc gcatttgcca gcggtaactt tattaaaaat 650

<210> 19
<211> 620
<212> DNA
<213> Actinobacillus pleuropneumoniae

<400> 19
gcaatacctg cactcgggcc gtctttcggc gttgcgccgt ccggcacgcg tacgtgaata 60

tcacgttttt catagaaggc atccgcactg cctaattgtg cggatcttgc acgtaccacc 120

atcattgccg cttgaatcga ttctttcatc acatcgccta atgaaccggt aaatgagaat 180

ttacctttac cgccgaccga agtggtttcg atggtgagta aatcgccgcc gacttccgtc 240

caagctaagc cggttacttc accgacacgg ttttggctgt ccattttacc gtaatcaaaa 300

cgttttacac ccaagtaatc ggcgatattg ccttcggtta ctgtaattga tttgactttt 360

ttgtccaaca ccagagtttt caccgcttta cgacagatct tagcaatttc acgttcaaga 420

ctacgcacac cagcttcacg agtgtagtaa cgaataatgc ttaaaatagc actatcttcg 480

atagttaatt cacccgcttt taagccgtta ttttcttgct gtttggcaat taagtggtca 540

cgggcaatgt gcattttctc atcttcggta taaccggaaa gacgaatcac ttccatacgg 600

tcgagtaatg ccggcggaat 620

<210> 20
<211> 272
<212> DNA
<213> Actinobacillus pleuropneumoniae

<400> 20

agcgaagcta ccgctcgaag taaaagaaaa actagatagc gagtttaaaa agttaaaagc 60

gatgccgcaa agttcttccg aagcgaccgt cgtacgcggc tatatcgatt gggtcttaaa 120

aatgccgtgg aacaaaaaat caactgtgaa aaaagacttg gcgaaagcgc aagaaatttt 180

agacaaagat cactatggtt cggagcgtgt taaagagcgc attgtcgaat accttgcggt 240

acaaagccgg ttaaacaaac ttaaaggccc ga 272

<210> 21
<211> 669
<212> DNA
<213> Actinobacillus pleuropneumoniae

<400> 21
agtacctttt caaagcttgg gttgcttttg caaaccaaga ttggattagc gtaatctcat 60

tacttgaaag taagctctct gagttaagta aaaaagactt agatttatat ttgcctgagc 120

tattgcttgc tcgagcttac tgtcaattaa atgactttac ctcctcacat aattgtttag 180

ttgcatttga gagacactct gctgcatttc cattgtctcg aatcgaaata gcacacctag 240

cacactctgc cggagcgcca ttgtctcgaa tcgaaatagc acacctagca tatgcaagac 300

gaaattatac aaaatgtatt gatcaactta ataaatgctt tgctaaagaa cttgacaatc 360

tacctgcaga gagtttagag gaatatgctc tttccttact taaaggaaat aacactaagg 420

aatttgaaaa attagtagaa agttctctaa gtgagaagtt taaagatcga actttcttta 480

aaaaagaata tgtttccttt cttgctaaaa atcagttatg gaaaaaatta gttaaatatg 540

ctagcaattg ggcattacaa gataaagaaa aattcaatta tccattaatg cttgctcatt 600

atagattagg agatattgca tatgtttatc agaatcatat aaaaccgact gaagaacatc 660

cctatgaat 669

<210> 22
<211> 277
<212> DNA
<213> Actinobacillus pleuropneumoniae

<400> 22
ggctttgcag tagcggaaac gcaaaatgta tcgtcggata aattaccgga aaatgtggcg 60

ccggcttata ccaacccgca taaagatatc ggtaacattc cggtgacttt ccctcatcaa 120

ccgccgcttg ttccgcatag tattcgtggt ttacaagtaa ctaaaaatac caaccaatgt 180

ttaggttgcc atgcgccgga agtatcaagc acgacgggtg ctccgcgtgt gcctaaaacg 240

cattttatgg atcgagacgg taaattgacg gaaggaa 277

<210> 23
<211> 807
<212> DNA
<213> Actinobacillus pleuropneumoniae

<400> 23
tggaagtgac tcgtctgcgt gatacgccga tcttaacttt tatgaacaaa ctcgaccgtg 60

atatccgtga tccgatggag ttactagatg aagttgaaag cgtcctaaaa atccgttgtg 120

cgccgattac ttggccgatt ggttgcggta aattatttaa aggcgtctat catattgcaa 180

aagacgaaac ctatctttat caatccggtc aaggttcaac cattcaagaa gtacgtatcg 240

taaaaggctt gagcagccca gaacttgatg ctgcggtcgg tgatgattta gcgaaccagt 300

tacgtgaaga attagaactt gtacaaggtg catcaaacga atttgatcac gaagccttta 360

tcaacggcga actcacaccg gtattcttcg gtaccgcatt aggtaacttc ggtgtggatc 420

atttcctaga cggtttaacc gaatgggcgc caaaaccgca agcacgtcaa accgatgtac 480

gtacggtcga atcaagcgag gaaaaattct ctggcttcgt gcttcctgtt aagatccaag 540

cgaatatggg cccgaaacac cttcaccgat tggcatttat gcgcgttggg ggaggcaaat 600

accgaaacac ggtcttcaaa ctcaaacacc tacgtatcgc cataagatgt ctgcgatttc 660

cagatgcctt aacctttatg gcgggtgaac gtgcacatgc tcgaagaagc ttacgcgcgg 720

cctatattat cggtttacat aaccacggaa caatccaatt cggtgacacc ttcactcacg 780

gtgaagtgat gaaatttacc gccttcc 807

<210> 24
<211> 402
<212> DNA
<213> Actinobacillus pleuropneumoniae

<400> 24
cacgcggtac ggatgtcatt ttacatttgc gtgaagcaga aaaagaattt ttaagcgaat 60

ggcgtttacg tgaaattatc ggtaaatatt cggatcacat cggcttaccg gttgaaatcc 120

aaaccaccga atatgatgaa gaaggcaaag caagcggtca aaaatgggaa aaaattaaca 180

aagcgcaagc actttggact cgttcaaaaa ccgaaatttc agacgaggaa taccaagagt 240

tttataaaca tttaagccac gattataatg actcgctcat ttgggcgcat aataaagtag 300

agggtaaaca agactatacc agcttgcttt atgtaccggc gaaagcacca tgggatctgt 360

tcaatcgtga tcaaaaacac ggcttaaaac tttatgacag cg                    402

<210>  25
<211>  551
<212>  DNA
<213>  Actinobacillus pleuropneumoniae

<400>  25
tatcttcgca tcggtgagcc gccatgttcg gttaaatgcc ctactaccca ttcgtcaata      60

cgatcagata ataacagcac ttcaatgcct ttcttattga acagttcaag gtgcgggcta     120

tttttcgccg ccacataact atcagcggtt aagaaataaa tcgctttctg accttctttc     180

attctgccga tataatccgc caaacttacc gtttgttcgc tagaatcagt ttgggtcgaa     240

gcaaaacgga ataatgacgc aacttgttgt ttattcgcaa aatcttcacc aacgccttct     300

tttaacacta aaccgaattc gttccagaag gtttggtatt tagcttgatc gtccttcgct     360

aatttttcca ataattgcaa tgcacgttta gttaatgccg cacgtagtga agcagtaact     420

ttattctctt gtaaaatctc acgtgaaacg ttgagcggca agtcattcgt gtctaataaa     480

ccccgcataa aacgtaggta attcggcata aacactttcg gcatcggcca taataaatac     540

acgctgtaca t                                                          551

<210>  26
<211>  281
<212>  DNA
<213>  Actinobacillus pleuropneumoniae

<400>  26
cacttagccc ccaaattagc agttcctaaa aaaaagcctc cctcaaagga agtaaaaaaa      60

gcgcctgaaa agatcatgaa aaaacatgag acaaagaaag atgtttctcc aacaaacaaa     120

agcagtcgta ctcagcagcc gatccaacaa ggaaatccaa atggtataaa tggaagcaaa     180

tcaacctatg gaaatgccaa agtcgatgcc tcccttggtg ctggttatgg caatgcaatg     240

cgagggccgt tgctctgata tttcagatga atctgatgat g                         281

<210>  27
<211>  735
<212>  DNA
<213>  Actinobacillus pleuropneumoniae

<400>  27
attatgaacc aattaaaaaa tgatcgttac ttaaaagcgt tactacgtga accagttgat      60

atgacaccgg tatggatgat gcgtcaagcg gggcgttatc taccggaata taaagcgaca     120

cgtgcggaag ccggcgattt tatggcttta tgtcgcaatg cagatttagc ttgtgaagtg 180

acgttacagc cgctacgccc gctacgccgc tatgcattgg atgcggcgat tttattctcc 240

gatattttaa ccgttccgga tgcaatgggc ttaggtttaa gtttcggcgc gggtgaaggg 300

cctaaatttg ctcgcccgat tgaaaataaa gcgcaggtgg acaacttacc gattccggat 360

ccggaaagcg aattacaata tgtgatgaat gcggtacgta ctattcgccg tgaattaaaa 420

ggcgaagtgc cgttaatcgg tttttcaggc agctcgtgga cattggcgac ttatatggtg 480

gaaggcggct caagcaaagc gttcaccaaa attaaaaaaa tgatgtatgc cgatccgaaa 540

attttacacg cattattaga taaattagcc gatagcgtga tcttatatct gaatgcacaa 600

attaaagccg gcgcgcaagc ggtgatggtg ttcgatactt ggggcggcgt gttggcgcat 660

aacgaatata aagagttctc gctacgttat atgcacaaaa tcgtggacgg tttaattcgt 720

gaaaacgaag gcaga 735

<210> 28
<211> 539
<212> DNA
<213> Actinobacillus pleuropneumoniae

<400> 28
tgcgtggtat ccacacgatg gaacacttat ttgccggctt atgccgtgat catttaaata 60

gtgaaagtgt agaaattatt gatatttcgc cgatgggttg ccgtaccggt ttctatatgt 120

cgttaatcgg tgcgccaagc gaagcggatg tggtttcggc gtggacaaaa tcgatggaag 180

atgctttaaa taaagtaccg gacgtgtcaa aaattccgga attaaacgaa tatcaatgcg 240

gctcttataa agagcattcg cttgaagaag cgcatcaaat cgctcgtgat gtgttagcca 300

agggtatcgg cgtaaaccgt aacgaagatt tagcgcttga tgagaaatta ttaaatccat 360

aatccttcaa aaaggcgtaa tgtttattac gtctttttct tcttaaaaat atattttaat 420

ctttgacaga cgcttatggc gtcttttagt tttttcagga aagtaagatg ttcaacaaaa 480

tttttagttg gtttgaagca agagtggaaa cttaccccga agcacccgcg aaaacgccc 539

<210> 29
<211> 104
<212> DNA
<213> Actinobacillus pleuropneumoniae

<400> 29
cgcagtgtaa gtttttgccc gattgcacga tgtagaagaa gtgcggttac tgatgaatcc 60

acgccgcctg ataaacctaa aatgacttcg tcatcgccta cttg 104

<210> 30
<211> 1201
<212> DNA
<213> Actinobacillus pleuropneumoniae

<400> 30
tagaaaaact ctgaagtcct tgagaatttc cgctcttcgc catagcctgt aacgcttggg 60

tcggcacgtg taataagctg ttggtcagtt tataacttag ctcgttaagg actttttcac 120

tatcttgtcc actggttaag gcaactaatg ctttcgctaa tagctcttga cgagtttgct 180

cggcatcttg gcgataacgt ttaatcaaat cgctggattg ctgttgtttc agccattcaa 240

agaacgcttt acattcttca tccacaattt gtgccgcttg ttctgcagct tgttctcgtt 300

gcgccatatt acgttgaatg atatgttgta aatcatctac gctgtaagaa tataccgcat 360

ccaattcgcc ggcattttcg tcaatatcgc gaggaacggc aatatcaatc aataacatcg 420

gatcaaactg gcgttgtttc tgtgcgattt ccaccatttc tttgctaatc agcatatccg 480

gactgccggt agaactgata acgatatccg cttggtttaa accgatttgc aatgcgctta 540

atgataaaat ttgcatcggc gtgtttaaac gctccgctcg ctccgctaac gtttccgcac 600

gttgcggcgt acggttggcg atcataatgt ttttcgcgcc gtgctgaatt aaataacgcg 660

cgactaattc gatggtttcg ccggcaccga ccaacaagaa acgcaattta ccgaaattat 720

cgaaaatctg gcgtgccaaa ccgcaagcgg cataagcgac cgataccgca cttgaaccga 780

tttccgtttc ggaacgcaca cgttttgccg tcgaaaaggt acgttggaac agacgggata 840

atttagtaga aatatggctg ttctgcgatt ggtaaaaatt ctcgctatat tgataagctt 900

gttttacctg gcctagaatt tgcggctcgc ccaagataag cgaatctaaa ccgcacgcta 960

ctcgcattaa atgacgagcg gcttccatat tttgtttgaa ataaatacat tcgcgtagtt 1020

cattatgttc aagttgatga atttcggcaa accattcaaa acattgttca cgccatgcga 1080

tgttatcttc atgttcttct cgcggcgtaa tttccgcatt atgaaaataa agctctgttc 1140

ggttacacgt tgaaagaatg acaacgcttt cagcaagtgc gcgggggttg aatttgctca 1200

a 1201

<210> 31
<211> 250
<212> DNA
<213> Actinobacillus pleuropneumoniae

<400>  31
agcaaagttt attccaacaa tcaaatctat tgattacgga ctattcatct attgcatttg      60

aaatggcatt tctaggaaaa caaacaatct attaccaatt tgataaagag gaatttagat     120

ctggaattca tacatatcaa caaggatact ttgaatacga gaaagatgga tttggtcctg     180

tagctgaaac attagatgat ttatttattc acctagataa attcgtaaac ggtgaaaatg     240

attcataaat                                                            250

<210>  32
<211>  364
<212>  DNA
<213>  Actinobacillus pleuropneumoniae

<400>  32
cgccacgctt atatacaaca accgccaccg tattggataa attcatactg cggctgtctt      60

tacacattgg cactcggatt ttttgcgaca tcggtaaatt atccaaaatc gtcatcggga     120

taccacggct ttccggccca aacataagga agtcgcctaa ttcataactc acttcactat     180

gattcggttc gcctttagtt gtcagcgcaa atagacgttt tggcttggcg cgttctagaa     240

aatcttcaaa attcttatat ttttgaatat cgacaaattc gtggtaatcc aaacctgaac     300

gacgtagttt tttgtcatcc cacgcaaagc ctaacggttc aatcatatgt aatctaaaac     360

cgca                                                                  364

<210>  33
<211>  486
<212>  DNA
<213>  Actinobacillus pleuropneumoniae

<400>  33
cgcttggtaa gcgattgccc gtgcggctcg cataagtaac cttcccatat tgtgctttaa      60

tgctgcttcg gtttccgcat cgtttaatgc cgcctaacct aaatcgctta acgcccaacc     120

gttgcgttta aataattctt ccacacgagg ccagttgttc gggcggctta cgtccattgc     180

gtttgaaagc gttgctaccg ttgcgttcgg ttcccattta cctgatttta aataacgcgg     240

cactgtgtca ttcgcattgg ttgatgcgat aaaacgttta attggtaaac ctaaggtttt     300

tgcgattaaa ccagcggtta agttaccgaa gttaccgctt ggtactgaaa ccactacatc     360

agaacgtttt gctttcggta attgtgctac cgcttcaaag taataacaaa cttgcgctaa     420

taaacggctg atattgattg agttttgcgg agttttaagc ccgatcgttt gacgtaattc     480

agcaat                                                                486

<210> 34
<211> 241
<212> DNA
<213> Actinobacillus pleuropneumoniae

<400> 34
attgtttccg atatccttga gttagtaaat cctgatttag cccatattca ttgtattcaa 60

ggcttaggcg ttggtgttgt tgatgcatgc acacaacgaa atataaagac tgctgtaacc 120

ttacatgatg cttggtggat ttgccctcgc caatttatga ttaaagctga tggtaagttc 180

tgtcatcaat ataaaattga taaaaatgaa tgtataaaat gtattggtag tacatcagaa 240

t 241

<210> 35
<211> 479
<212> DNA
<213> Actinobacillus pleuropneumoniae

<400> 35
tactaaacga ggatttttca cttgcggacc ggcaagcgaa ataatacggt cggtaaagag 60

ttcgcctgta gtaaataatt taccgatagc aatcacatct tgataattta agtgccaaac 120

ttgtttggtc gcccctacgg gatcaacaaa gtggatatgc gtaccgacta aaccggcagg 180

atgaacaccg ctaaacgact taattgtgat accttcaatc gccggactta acgggatatt 240

gctgtccgcg tctttacata agtaaaccgg tttttgaccg ttaaataaac gggttaaaac 300

agttaaaccg tctttaaaat cggtttcgta ctcttttaaa accacttccg gatccgccgc 360

taacggattg gtatccattg cattgacaaa gattgatgac ggaatcgcgt ccaatgcggg 420

aaccttactg aacggacgag tacggaatgc agtccataaa cccgattcga taagattttt 479

<210> 36
<211> 380
<212> DNA
<213> Actinobacillus pleuropneumoniae

<400> 36
tccacttctt ccgctaccgc tgcagccggc acttcaattt cattttgtgc gattaaaccg 60

ttgattactt ggtttttaac gcgtgcggtt actgcgtttt taagttcacg ttgcatattt 120

ttcttaattt ccgcacgtaa atcttctaca gtttttgctg aaccgaattt tttcacgaat 180

tcttcggtta attcaggtaa tacgatattt tctactttct taagtgtaat tgcgaatttc 240

gccgctttac cttttaagtt ttcagcgtgg tattcttcag ggaaagtaac atcgatatcg 300

aattgttcgc cggctttgtg accaacgata ccttctttaa aaccagggat catacgactt 360

tgacccattg ctaaagtgaa 380

<210> 37
<211> 498
<212> DNA
<213> Actinobacillus pleuropneumoniae

<400> 37
tgcaaaacgt cgtgttcaag taggtttatt actttcaacc gtaatcggta ctaacgaatt 60

aaaagttgat gaaaaacgtg ttgaagaaac gattgcagaa atcgcttcag cttacgaaca 120

accggcggaa gttgttgctc attatgcgaa aaaccgtcaa ttaaccgaaa atatccgtaa 180

cgtagtgtta gaagagcaag cggttgaagt tgtacttgcg aaagcaaaag taactgaaaa 240

agcgacttct tttgatgaag taatggctca acaagctcaa ggctaattct caaacataaa 300

gctctccggt ttggagggct tttaattagc attttataag cggttgattt taggaaaaaa 360

tttgcaaaaa tttctctaaa aatgaccgct ttctttttaa caagaagaga atttagtcat 420

ggatttaaca tttactcaac agctccaaca atttattgcg gcccatccta ttttaatttt 480

tgcttggatt gccttagt 498

<210> 38
<211> 604
<212> DNA
<213> Actinobacillus pleuropneumoniae

<400> 38
attatcagga agatgttgat tattatttaa tggcaaaacc aggaatgacg gggctttggc 60

aggttagtgg aagaaataat gttgattata agactcgagt atatttcgat gcttggtatg 120

taaaaaactg gtctctttgg aacgatattg ctattttatt taaaactatc aatgtattaa 180

ggagccgaga aggagcttat taatcctata gggctagagt aacttactat gcaatttgcc 240

ctttacttta taagccggct ggggtaaaat gtactacctt ttttagttct atgtcgccaa 300

aattgtgtgc gactttattt aaacatatat ttcctgaggt gatggcatat gccagtaatt 360

aaagttcgtg aaaatgaatc atttgacgtt gcattacgtc gtttcaaacg ctcttgcgaa 420

aaagcaggtt tattagcaga agttcgcgct cgtgaattct atgaaaaacc aactacaatt 480

cgtaaacgtg aaaaagcatc tttagctaaa cgtcacgcta aacgtaatgc tcgtgaaaat 540

gcacgtaaca ctcgtttata ctaattagca gtcttgctat ttaggcttaa attaactaaa 600

accg                                                              604

<210>  39
<211>  410
<212>  DNA
<213>  Actinobacillus pleuropneumoniae

<400>  39
cagccgagcc tatattgaat tagcggcaaa agtcgcttcg gagctatact ggcaaggttc      60

ggttatcccg tctgaaatta tgattcgcga agtaaaataa gttttaataa ccacgaaaac     120

acaaagaaca caagcggtag aatttgcaga aaaatctgca aatcctaccg cttttttatt     180

agtacgattc gctgttggac tgctatttga tttggtttgt caggatatta tgttattgta     240

atgaaatgtt agtgaattat ttttattaat ttgaaaggaa acaaaatgaa aataaaaaaa     300

cgttacattg cgctgttggt cttaggtgtc gttatcagct atgcctggta tcaaaattat     360

caatgggaac agctgatgtt aagcgggtat tgtgaaaagg acggaagtta               410

<210>  40
<211>  330
<212>  DNA
<213>  Actinobacillus pleuropneumoniae

<400>  40
ggttaagcct aaccgcttat taatgtcttt taatagctgt aaaatcgatt gtgtagtcgc      60

cggatctaat gcacttgtcg cttcgtcaca taataaaaca tgcggatcac ttgccaacgc     120

acgggcaatt gcgacacgtt gcttctgacc gccggataaa ttgctcggat aaacatcttt     180

acgttcggtt aagccgacca attcaatcaa ttcgtttact ttcttctcga tctgatcttt     240

cggcgtattg ctcaacgtaa gcggtaatgc gatattttca tataccgtat gtgatgccaa     300

caaattaaag tgctggaaaa tcatcgcaat                                     330

<210>  41
<211>  952
<212>  DNA
<213>  Actinobacillus pleuropneumoniae


<220>
<221>  misc_feature
<222>  (528)..(528)
<223>  n is a, c, g, or t

<400>  41
agtaacgctt ccatttttgc tgaaaaatca cgaccaagaa taatctcatc accgtgcaaa      60

acactactta cggcatggta tcttgcccat ttttgcttta attcgggact ttcgcataac 120

ccgtcgatga acgcatcatc aacttttttc ccgtccataa aagcggaaag ggtttcattt 180

tgttgcataa taaactccga ttgaatctta aatctgttgc attagcggat tgacctttgc 240

atcaatcgct tctcttgctc taaagatacg cgaacgaacc gtaccgacgg gacattgcat 300

gacttccgca atttcttcat aacttaaacc ttccaactca cgcaaagtaa ttgccgtttt 360

gagttcttca ggtaaatttt caatcgtgtc aaaaacgaca cgttttaatt cttcggatag 420

caccacattt tccggtgtat cactctcacg aagttgaccc ccaacatcaa agctttccgc 480

atcttccgcc aaaatatctt ctttaggagg acgtcttccc aaagcggnta aatgattttt 540

agcagtgttc accgcaatac gataaagcca cgtataaaag gcgctctctc cacggaaaga 600

ctctaatgaa cgatacgctt taataaaaga ttcctgcaca atatccggaa tatcatcacg 660

tgaaacatag cgagttaata aacctgccac tctattctga taacgtacga cgagtaaatt 720

aaatgctttt ttatcacctt tctgtgcacg ttcaaccaat tcttgatcgg ctaccagctc 780

actcatctat ttgcgttctc ctaatctatt tcgcatattc cttattgttc tcattccgta 840

catcctttag tgtgtaacaa aaatgaaaag ttcatataaa gaataaatta ctcaattatt 900

tacaaatact ttaataaaat tcaataacaa gactaaggat gctacaaata tt 952

<210> 42
<211> 1196
<212> DNA
<213> Actinobacillus pleuropneumoniae

<400> 42
aatattttta tgagcgtatt aaagatgaag acaccaatcc gctatccgaa caagagctaa 60

atcaatttgc gatggcatta cgcgtcgaat tacaaaataa accgaatgat gccaaaggct 120

ggtttatgtt aggtcaaatc ggcatggcga aagatgacgg gcaattagca ctggaaagct 180

acgaaaaagc cagcaaactg gacccgaaaa atctgcaata taaaggcagc tacgctcaag 240

tgctgatgtt ctcgcaagat caagcggata aggacagagg taaagcggtg ctaaaagaaa 300

ttttacgtga agatcatacc aatttagatg cgctcagctt actggctttc agttcttttg 360

aagaacaaga ctataaaatg gcggcaatga cttggggcat gatgctaaaa ctgattcctg 420

aaggcgaacc gcgccgcgct acagtagaaa aaagtatcaa tatggcaatg tcgatgttgc 480

aagagcaaga aagcaaacaa ccggctaaag cggaagagaa aaaataataa cattttagcg 540

aagctaaaaa ctgatgtcgg gcgggtttat acccgcccta aaattgcaaa aggataaata 600

atgttaagta aagagacatt aaaaatcggt ttggtttccg tttcggaccg tgcctccggc 660

ggcgtttacc aagaccaagg tattcccgaa ttacaagcgt ggttggaatc cgtattaacc 720

gccccgtttg aagtggaaac acgtttaatt ccggacgagc aagctatcat tgagcaaaca 780

ttaattgaat tggtggatac gcaccactgc catttagtct taaccaccgg cggtaccgga 840

ccggcaaaac gagacgtcac gccggatgcg acccttgcgg tggcacatcg tgaaatgccg 900

ggcttcggcg agcaaatgcg ccaagtcagc ctgcacttcg taccgaccgc gattctttct 960

cgccaagtcg gtgttattcg tcacgattcg ctaattttaa atttaccggg gcagccgaaa 1020

gcaattaaag aaactcttga aggggtaaaa gacgcacaag gtaatacgct ggtacgaggc 1080

attttcagcg ctgtgccgta ttgcttacaa ctattaagtg atatttatat cgacactcgc 1140

tctgaaaatc tgcgaaagtt tccgtccgaa atcagcaaga agataatctt taaaac 1196

<210> 43
<211> 601
<212> DNA
<213> Actinobacillus pleuropneumoniae

<400> 43
cgctaaacgt gcatatgttg gtttaggtag caaacagtat ggtgaagtaa catttggtcg 60

tcaattaaca attgctgatg attacggtca aacagatgat tacgaatacg gtatcgtacc 120

atcatatatc gcaacttcag gtaaccaagt gatccgttat gactataaag gtattgaagg 180

tttacaaatt ggtgcaaact ataatttcgc tgaaaaacgt aatgcgagag gtgaagtttt 240

agtaaatgaa ctcaaaaatg catatggttt aggtgcggta tatgaaacgc aattaggtgg 300

taacacattt aatgttgaag gtggtatcgg tcgttctaac tatgcaacag gtacaaacca 360

caaacattac caagatggtt atgaattagc attaggttat gctattggtg actttaaatt 420

agtttccgat ttcggttata aatatgagaa aaatggtgca gcgcgtacta aagcattctt 480

tgtagctcca ggcttccaat accaagttat ccctgcttca cgtatctatg gtaactattt 540

atatgagcgt actgaagtat cagattcaga tgtaaaaggt aaaactcatg gattcttatt 600

a 601

<210> 44
<211> 266
<212> DNA
<213> Actinobacillus pleuropneumoniae

<400> 44

taaaaataac tacggtccga tgttacgtgc tgcgttagag aagatcgaga aggaaggtta 60

agaaaatggg tttaaaaaat ctttttgaaa agatggaacc tgcgtttcac aaaggtggaa 120

aatatgagaa atggtacacg ctttttgaag cgacatatac cattctctat acaccaggta 180

cagtgactcg taaagattca cacgtacgtg atgcgttaga ttctaaacgt atgatgatta 240

tcgtatggtt agcgttattc ccggca 266

<210> 45
<211> 1065
<212> DNA
<213> Actinobacillus pleuropneumoniae

<400> 45
aactaaggat agatatgtta cagaataaga aaattgcagc agttattcca acttttagag 60

tgcttgctca gattgaaacc gttttgaaaa gaatacccga gtattttgat atgatttatg 120

tcgtggatga taaatgtgat caaggtagtg gtaaatttgt tcaagaacat attatggatt 180

ctcgagtaaa agtgcttttt catgaagtga atcaaggtgt tggaggagct gttattactg 240

gttataaaca agcaattatc gatcagatgg atgtagtggt taaaattgat ggtgataacc 300

aaatggatcc ttatttggct gaaaaattta ttttaccaat tgttaatgga gatgcagatt 360

atacaaaagg aaatcgattc tttaaccttt cagacgtagc ggatatgccg aaagtgaggt 420

tttttggtaa tattgctctt tcttttttaa ccaaagcatc atctggttat tggaaaattt 480

ttgatccgac aaatggttat acggcaatta gtgttaaagt actaaaaatg ttggctttag 540

acaagattca aaagcgttac tttttgaaa gtgatatatt attccgattg aatatgatct 600

cagctaaagt attagatgtg ccaatggttg cagtatatgg agatgaagta tccaatttaa 660

aaattagcaa aatattcttt ccatttctaa aaggaaattt atctaatttt tcaaagagaa 720

ttttctataa atatttcttg caagatttta atgttgcttc tgttgaatta gtatttggta 780

cagtaatgtc attattcgga atattatttg gactatataa ttggattgct aatgcaatta 840

ataatataga aacaccaaca ggaaccatta tgatttctgc attgtttatt ttagtaggaa 900

ttcatttatt actctctttt atatcttatg atgtaaataa ttatccaacg gaatcaatta 960

gtaaaaattt agatgaataa tcccatttgt tctgttattg tttgttatga accaactaag 1020

aaaatatttt ttttaatcaa ctctctaatt aagcagaatg tatat 1065

<210> 46
<211> 155

<212> DNA
<213> Actinobacillus pleuropneumoniae

<400> 46
ggaaggctta aaacagctgt ttcagggaag tcaccccgtc aaaaaactga tacgaggcgt 60

aggtttatcc gcaaccaatc aactaccggt attaaagaaa ttattgattg ctcaggcggt 120

gggtatttga cttgtctgtt ttttagtcag ataat 155

<210> 47
<211> 771
<212> DNA
<213> Actinobacillus pleuropneumoniae

<400> 47
tttttggcgt tttagaaaag tcgcaaattt ttccggctct ttttgttgat aggcgaaagt 60

catcgtggac tcctttaata agaaagtgga agatttgttc gaattacaat caatcgaagt 120

ttatttgtca aaataaaaaa atgaaatctt tgaactactc cacaaattaa ttgagcaagc 180

ggttaaattt acaaattttt ttgcaaatcc aaccgctttc cattagaatt ataagacttt 240

tggggctgat tctggattcg acgggattag cgaagtccaa ggtgcacgtc gaggtgcggt 300

aggcctcgta aacaaaccgc aaaaaaatag tcgcaaacga cgaacaatac gctttagcag 360

cttaataacc tgctcatagc cttatcgcct cagcttccgc tcgtaagacg agggcaacga 420

taagtcaccc aaaacgagat cgtgtggacg ccgccgtttg aggatcgaaa cactaaattg 480

aatcaaacta gcttatttct tgcgtgtctg tccgctggag gtaagtgaaa ttaaagacca 540

gactaaacgg actaaacgtg tagtgctgaa gatggagtaa tttcggacgg gggttcaaat 600

ccccccagct ccaccaaatt tgtatacttt taataggtta tttaaaatga aattccacca 660

aaaactaaat agaactgcca aaactgcagt ggctattgca cttagtagtt ttggtgtatt 720

aggtatgtct tcattagata atgtcactca atctattgaa aatataagaa t 771

<210> 48
<211> 313
<212> DNA
<213> Actinobacillus pleuropneumoniae

<400> 48
ggtcggcgat aagatcgtaa gtgtcggtca ggataacgat gagattgaag atgttatcgg 60

ctggcgttta gatgatgttg tcgataagat catacgtact aatggtacta aagtccggtt 120

cgaaattgaa ccggaaaaag gcggtgaaac gaaagttatc actttggttc gagataaagt 180

ccgtttagaa gacagtgcgg cgaaattaac cgttgataaa attaacggta aaaatgtcgc 240

ggtaattaaa atttcaacgt tttacatcgg tttgaccaat gatgtgcgta aattattaga 300

tgaaatgtac act 313

<210> 49
<211> 225
<212> DNA
<213> Actinobacillus pleuropneumoniae

<400> 49
   ttcttaattt caggccattt tttatcttta agatagagat taattacatc gtttttaaca 60
   cgttgttccc atagctgatt ggcttcttca accgttgcgg gaaacgccgc cttttcacga 120
   tcattttcga tttgatccga cccttttaaa ttcggctctt tatctaataa agacaacgca 180
   tacttataac gtccataacc gacgtttggt cattaaatca tagat 225

<210> 50
<211> 1045
<212> DNA
<213> Actinobacillus pleuropneumoniae

<400> 50
attcgtgccg cattagtgaa atacgatccg aaaaatgccg aaagctataa ccaaaatgct 60

aaagtttatg cagaaaaagt aaaagcaatt gccgaaccgt tacgccaacg tctatccgtt 120

attccggaag cacaacgttg gttagtaacc agtgaaggtg cattcagtta tttagcacag 180

gactatcaat taaaagagct ttatctctgg gcaattaatg ctgaagagca aggctcgccg 240

caacaagtga aaaaagtaat tgacggtgta aaagccaaca atatccctgt tgttttcagc 300

gaaagcaccg tatcggataa accggctaaa caagttgcta aagaaaccgg tgcattatac 360

ggcggtgtgt tatatgttga ttcactttca actaaggatg gtgctgttcc aacttattta 420

gatctattaa aagtcactat aagtacaatt gtggacggtt ttgaaaaaag taaaaaataa 480

catggtatcc attcctactt ctatttccgt tgaaaagcta agtgagctaa gtgtccgtta 540

taataacggg catttagcct tatatgacgt atccttccaa ctgcaaaacg gtacgatttg 600

cgccctaatc ggcgtaaacg gcggcggtaa atccacacta tttaaaagtt taatgggatt 660

agttaaacct caaaccggta caattctgtt aaaccgaatg ccgattcaac aagcattgaa 720

acaaaatctg gtgtcttatg taccgcaaag tgaagaggtc gattggcaat ttccggtttc 780

cgtttacgat gtcgttatga tgggacgtta cggttatatg ggattgctac gccgtccttc 840

atctcttgat aaacaaaaag tgatgcaagc gatggaacga gtggatattt gccacttgca 900

gcatcgccaa atcggtgaac tttccggcgg gcagaaaaaa cgcgtgtttc ttgctcgagc 960

attagcgcaa gaaagccaaa ttattttgct tgacgagccg tttaccggtg ttgatgtaaa 1020

aaccgaaaat gccattggtg gaatt 1045

<210> 51
<211> 263
<212> DNA
<213> Actinobacillus pleuropneumoniae

<400> 51
tttttagata gcgtgaaaaa cttcttctct aacttaggta aacactaaag attaaaaagg 60

cttagcgaat ttgctaagcc ttttatttta ggcatagtag acaaaaaggt tggtaaaaag 120

tgggttaaag ccttatagta caaggcttta acctaccttt tgaaatatga ataaaaactt 180

gccctttttg tcaatcttcc aaacacaaaa gtatggctta aaaataagct tcaacgttat 240

aaatgttcag cttgcaataa aat 263

<210> 52
<211> 1195
<212> DNA
<213> Actinobacillus pleuropneumoniae

<400> 52
cttccgatga tacggaaatt ttagtttttt taactcaaga tgctttattt gcaagttcag 60

attctctaga agaattaata aaacctttcc aagatcctga agtaggggct gtttatggta 120

ggcagttacc tcacaaagat gcgactctat tagctgccca tgccaggtta tttaattatc 180

ctcctaaaag tataattaag tcgaaagaga gtatttcgga gttaggatta aaaacagcat 240

ttatctctaa ttcttttgcc gcataccgct gttctatttt tgaagaactc ggaggttttc 300

ctgagaaaac gattttagcg gaagatatgt acttggctgc taagataatc ttgaatggtt 360

ataaaattgt ttataatgcg gaggcaaaag tctatcattc ccataactat tctttgattc 420

aagagtttca gagatatttt gatactggag tattccaaaa agagcaatct tggattagaa 480

aagaatttgg tgaagcaaat aatgagggta aaaagtttgt atgttctgag attaagtatt 540

tattgaagaa taatttttta ctattgtcga aagctatatt tcacactatg tttaagtttt 600

tagggtttaa attaggtttg aattatgaga aattacctag atggttatgt ataaaattta 660

gtatgcataa gaactattgg aaataaatca tgaaaaggca gatattatct aaatatacgt 720

tagctataag tgattttatt agtttttctc tatcatttat tctatcttta gtattattaa 780

attattcaat tgagaggttt gatgcttatc tcccacttga tcaagttaga gaaagaatga 840

ttatccactt atcgttaggt tttataggtg ttatatggtt ttggattagg cttcgccact 900

atacttatcg taaacctttt ggtttgaact aaaagaagtg atcagaacac ttattattct 960

tgctatattg agctagctac tatagccttt tcaaagcttt atttttctcg ctattatggg 1020

gtttaacttg ggtgttacaa ttcttctttg ttcctattgt tcgtatttaa ctaaaaaaat 1080

cttaattgat acgggactat atattaagaa tacagtatta ttggtggagg gaataatgcg 1140

attgatgcct ataaagcctt aactagtgag tcttatttag ggttaaaagt aaaat 1195

<210> 53
<211> 288
<212> DNA
<213> Actinobacillus pleuropneumoniae

<400> 53
ggatctttca ttacctttat caccataccg ctaccatctt cgagtaacca agagaagcat 60

ccgatttcgg atcataagca ccgatacgaa cttgttcgac taaaacaacg ctatcacgaa 120

cgggatcgta agcgattaat gccgccgctg cccctataat cagtaattca cgcacgattt 180

caccgctcat ttcaccggag aatagcttat gtcggaaata cattttcttt aattcaaaat 240

gccctttata aacggtttct tctttaatca tgttgaagat actgttga 288

<210> 54
<211> 290
<212> DNA
<213> Actinobacillus pleuropneumoniae

<400> 54
gcggacaata caatctcctc cattgtcctc ggcttcattc tacactataa tccgttcaat 60

ccggtgcaac gcacggctta taagaggaac gccaatatat tctggtacac gttgtcagcc 120

gatatcaagc ctatcaatgg gtgtgtgaag gcaaaatcga taatgttatc gcagtggtcg 180

gtttacaatg gttacaactt aattatcaaa aatatcgagt gtaatgagcg ttttccgata 240

acgacgaata tatttgcccc tctttaatga gggcaaattt tttgaagtat 290

<210> 55
<211> 47
<212> DNA
<213> Actinobacillus pleuropneumoniae

<400> 55
cgctcatcac caaacacttg aggttctaag agttttacat ccggaat 47

<210> 56
<211> 241
<212> DNA
<213> Actinobacillus pleuropneumoniae

<400> 56
cgcacctaga tatcgggata cgcgcggtca attaggcatc tataccgaaa cgggtttatt 60

atcagttcaa aataacgtat taccgcaact tggtaaaaag taatcttgat ctaatgcttt 120

agaaaactta gaattaatca aaagccccat agtttatgct ttggggcttt aattttttca 180

tcagttttta aattaagcgg tcaaaaatta agaatgtttt gcaaatgaaa ttagttgaaa 240

t 241

<210> 57
<211> 19
<212> DNA
<213> artificial sequence

<220>
<223> synthetic primer

<400> 57
cgactacaac ctcaagcta 19

<210> 58
<211> 18
<212> DNA
<213> artificial sequence

<220>
<223> synthetic primer

<400> 58
cgaccattct aaccaagc 18

<210> 59
<211> 28
<212> DNA
<213> artificial sequence

<220>
<223> synthetic primer

<400> 59
cgccatgtcg accattctaa ccaagctt 28

<210> 60
<211> 27
<212> DNA
<213> artificial sequence

<220>
<223> synthetic primer

<400> 60
cgcctagtcg actacaacct caagctt 27

<210> 61
<211> 9
<212> DNA
<213> artificial sequence

<220>
<223> synthetic primer

<400> 61
gtcgaccct 9

<210> 62
<211> 9
<212> DNA
<213> artificial sequence

<220>
<223> synthetic oligonucleotide

<400> 62
gtcgacagg 9

<210> 63
<211> 19
<212> DNA
<213> artificial sequence

<220>
<223> synthetic primer

<400> 63
gacaagatgt gtatccacc 19